(19) Europäisches Patentamt
European Patent Office
Office européen des brevets

(11) Veröffentlichungsnummer: **0 075 207**
**B1**

(12) # EUROPÄISCHE PATENTSCHRIFT

(45) Veröffentlichungstag der Patentschrift: **05.02.86**

(21) Anmeldenummer: **82108292.2**

(22) Anmeldetag: **09.09.82**

(51) Int. Cl.⁴: **C 07 C 93/06**, C 07 C 149/24, C 07 C 93/14, C 07 D 295/08, C 07 D 211/46, C 07 D 211/52, A 61 K 31/13, A 61 K 31/445, A 61 K 31/495

(54) **Aminopropanolderivate von 2-Hydroxy-beta-phenyl-propiophenonen, Verfahren zu ihrer Herstellung und diese enthaltende therapeutische Mittel.**

(30) Priorität: **18.09.81 DE 3137178**
**17.07.82 DE 3226863**

(43) Veröffentlichungstag der Anmeldung: **30.03.83 Patentblatt 83/13**

(45) Bekanntmachung des Hinweises auf die Patenterteilung: **05.02.86 Patentblatt 86/6**

(84) Benannte Vertragsstaaten: **AT BE CH DE FR GB IT LI NL SE**

(56) Entgegenhaltungen:
**EP-A-0 074 014**
**DE-A-2 001 431**
**US-A-3 812 129**

(73) Patentinhaber: **BASF Aktiengesellschaft, Carl-Bosch-Strasse 38, D-6700 Ludwigshafen (DE)**

(72) Erfinder: **Franke, Albrecht, Dr., Mandelring 11, D-6706 Wachenheim (DE)**
Erfinder: **Mueller, Josef, Dr., Karl-Otto-Braun-Strasse 16, D-6700 Ludwigshafen (DE)**
Erfinder: **Lietz, Helmut, Raiffeisenstrasse 15, D-6730 Neustadt (DE)**
Erfinder: **Wiersdorff, Walter-Wielant, Dr., Blockfeldstrasse 15, D-6704 Mutterstadt (DE)**
Erfinder: **Hege, Hans-Guenther, Dr., Dinckelackerring 29, D-6730 Neustadt 14 (DE)**
Erfinder: **Mueller, Claus D. Dr., Odenwaldring 84, D-6806 Viernheim (DE)**
Erfinder: **Gries, Josef, Dr., Roemerweg 43, D-6706 Wachenheim (DE)**
Erfinder: **Lenke, Dieter, Dr., Kekuleplatz 1, D-6700 Ludwigshafen (DE)**
Erfinder: **Von Philipsborn, Gerda, Dr., Naechstenbacher Weg 35, D-6940 Weinheim (DE)**
Erfinder: **Raschack, Manfred, Dr., Donnersbergstrasse 7, D-6714 Weisenheim am Sand (DE)**

**EP 0 075 207 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

## Beschreibung

Gegenstand der vorliegenden Erfindung sind neue Aminopro-panolderivate von 2-Hydroxy-β-phenyl-propiophenonen und ihre physiologisch verträglichen Säureadditionsverbindun-gen, ihr Herstellungsverfahren sowie diese enthaltende therapeutische Mittel, die als Antiarrhythmika verwendet werden können.

Aus der DE-OS 20 01 431 und der EP-A-74014 ist bekannt, daß die n-Propyl-amino- und 1,1 - Dimethylpropylamino-, n-Butylamino-, sec.-Butylamino- und tart.-Butyl-amino-propanolderivate des 2-Hydroxy-β-phenyl-propio-phenons antiarrhythmisch wirksam sind. Das gilt besonders für das 2-(2'-Hydroxy-3'-n-propylamino-propoxy)-β-phenyl-propiophenon-hydrochlorid, das unter der Bezeichnung Propafenon als Antiarrhythmikum bekannt ist. Aufgabe der Erfindung ist es, demgegenüber verbesserte Antiarrhythmika zur Verfügung zu stellen.

Es wurde nun gefunden, daß Aminopropanolderivate von 2-Hydroxy-β-phenyl-propiophenonen der Formel I

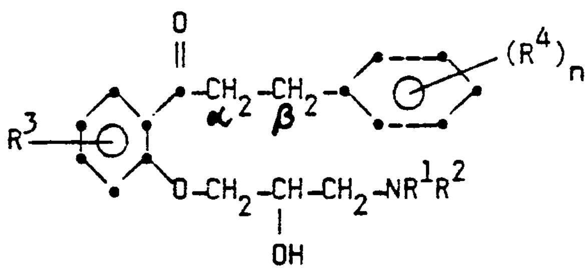

I

.

in der

$R^1$ und $R^2$ gleich oder verschieden sind und Wasserstoff-atome, Alkyl-, Cycloalkyl-, Alkenyl-, Alkinyl- oder Hydroxyalkylreste mit jeweils bis zu 8 C-Atomen, Alkoxy-alkyl-, Alkylthioalkyl- oder Dialkylaminoalkylreste mit WK/Kl jeweils bis zu 9 C-Atomen, oder Phenylalkyl- oder Phenoxy-alkylreste mit bis zu 8 C-Atomen im Alkyl und gegebenen-falls im Phenylrest durch Alkyl oder Alkoxy mit jeweils bis zu 3 C-Atomen substituiert bedeuten oder $R^1$ und $R^2$ zusammen mit dem sie verbindenden Stickstoffatom einen 5 bis 7-gliedrigen gesättigten heterocyclischen Ring bilden, der gegebenenfalls durch einen oder zwei Phenyl-und/oder Hydroxyreste substituiert sein und ein Sauer-stoff- oder Stickstoffatom als weiteres Hetaroatom im Ring enthalten kann, wobei ein zusätzliches Stickstoffatom durch einen Alkylrest mit 1 bis 3-C-Atomen oder einen Phenylrest substituiert sein kann, $R^3$ ein Wasserstoffätom, einen Alkylrast mit bis zu 3 C-Ato-men, ein Fluor-, Chlor- oder Bromatom, eine Hydroxygruppe oder eine Alkoxygruppe mit bis zu 8 C-Atomen ist, $R^4$ ein Wasserstoffatom, einen Alkylrest mit bis zu 3 C-Ato-men, ein Fluor-, Chlor- oder Bromatom, einen Alkoxyrest mit bis zu 3 C-Atomen oder den Rest $NR^5R^6$ bedeutet, worin $R^5$ und $R^6$ gleich oder verschiaden sind und Alkylreste mit bis zu 8 C-Atomen oder zusammen mit dem sie verbindenden Stickstoffatom einen heterocyclischen Ring darstellen, ist, wobei jedoch die Reste $R^1$, $R^3$ und $R^4$ nicht gleichzei-tig Wasserstoffatome sein dürfen, wenn $R^2$ einen Alkylrest mit 3 bis 5 C-Atomen bedeutet, und n die Zahl 1, 2 oder 3 ist, und ihre physiologisch verträglichen Säureadditionssalze wertvolle pharmakologische Eigenschaften aufweisen.

Von den Verbindungen der Formel I sind diejenigen beson-ders hervorzuheben, in denen $R^3$ Wasserstoff und n die Zahl 1 oder 2 bedeutet. Die Gruppe $NR^1R^2$ ist vorzugsweise ein Piperidin-, Piperazin-, N-Methylpiperazin-, Morpholin-oder Diisopropylaminorest. Weiter sind für $R^1$ und $R^2$ Propyl-, Butyl-, Alkoxyalkyl und Hydroxyalkylreste zu nennen, wie n-Propyl, Isopropyl, n-Butyl, Isobutyl, tert.-Butyl, Methoxymethyl, Methoxyethyl und Hydroxymethyl und Hydroxyethyl. $R^4$ ist vorzugsweise ein Wasserstoffatom, eine $C_{1-4}$-Alkoxy-, eine Dimethylamino- oder Diethylamino-gruppe.

Außer den in den Beispielen angegebenen Verbindungen seien beispielhaft genannt:

2-[2'-Hydroxy-3'-(2-hydroxyethylamino)-propoxy]-β-phenyl-propiophenon,
2-[2'-Hydroxy-3'-(2-ethoxyethylamino)-propoxy]-β-phenyl-propiophenon,
2-[2'-Hydroxy-3'-(2-n-propoxy-1-methyl-ethylamino)-propoxy]-β-phenyl-propiophenon,
2-(2'-Hydroxy-3'-propargylamino-propoxy)-β-phenyl-propio-phenon,
2-[2'-Hydroxy-3'-(4-hydroxybutylamino)-propoxy]-β-phenyl-propiophenon,
2-(2'-Hydroxy-3'-di-n-propylamino-propoxy)-β-phanyl-propio-phenon,
2-[2'-Hydroxy-3'-(N-methyl-N-propylamino)-propoxy]-β-phenyl-propiophenon,
2-(2'-Hydroxy-3'-pyrrolidino-propoxy)-β-phenyl-propiophenon, 2-(2'-Hydroxy-3'-cyclobutylamino-propoxy)-β-phenyl-propio-phenon,
2-(2'-Hydroxy-3'-cyclopentylamino-propoxy)-β-phenyl-pro-piophenon,
2-[2'-Hydroxy-3'-(1-methyl-2-phenyl-ethylamino)-propoxy]-β-phenyl-propiophenon,
2-[2'-Hydroxy-3'-(2-diethylaminoethylamino)-propoxy]-β-phenyl-propiophenon,
2-[2'-Hydroxy-3'-(3-diethylaminopropylamino)-propoxy]-β-phenyl-propiophenon,
2-[2'-Hydroxy-3'-(2-piperidinoethylamino)-propoxy]-β-phenyl-propiophenon,
2-[2'-Hydroxy-3'-(2-morpholinoethylamino)-propoxy]-β-phenyl-propiophenon,
2-[2'-Hydroxy-3'-(4-ethyl-1-piperazino)-propoxy]-β-phenyl-propiophenon,
2-[2'-Hydroxy-3'-(2-methylaminoethylamino)-propoxy]-β-phenyl-propiophenon,
2-[2'-Hydroxy-3'-(3-hydroxy-piperidino)-propoxy]-β-phenyl-propiophenon,
2-[2'-Hydroxy-3'-(4-phenyl-piperidino)-propoxy]-β-phenyl-propiophenon,

2-[2'-Hydroxy-3'-(4-hydroxy-4-p-chlorphenyl-piperidino)-propoxy]-β-phenyl-propiophenon,
2-(2'-Hydroxy-3'-n-propylamino-propoxy)-5-methyl-β-phenyl-propiophenon,
2-(2'-Hydroxy-3'-n-butylamino-propoxy)-5-methyl-β-phenyl-propiophenon, .
2-(2'-Hydroxy-3'-isopropylamino-propoxy)-5-methyl-β-phenyl-propiophenon,
2-(2'-Hydroxy-3'-tert.-butylamino-propoxy)-5-methyl-β-phenyl-propiophenon,
2-[2'-Hydroxy-3'-(2-methoxyethylamino)-propoxy]-5-methyl-β-phenyl-propiophenon,
2-[2'-Hydroxy-3'-(4-hydroxybut-2-ylamino)-propoxy]-5-methyl-β-phenyl-propiophenon,
2-[2'-Hydroxy-3'-(4-hydroxy-4-phenyl-piperidino)-propoxy]-5-methyl-β-phenyl-propiophenon
2-(2'-Hydroxy-3'-piperidino-propoxy)-5-methyl-β-phenyl-pro-piophenon,
2-(2'-Hydroxy-3'-n-propylamino-propoxy)-4-methyl-β-phanyl-propiophenon,
2-(2'-Hydroxy-3'-n-butylamino-propoxy)-4-methyl-β-phenyl-propiophenon,
2-(2'-Hydroxy-3'-isopropylamino-propoxy)-4-methyl-β-phenyl-propiophenon,
2-(2'-Hydroxy-3'-tert.-butylamino-propoxy)-4-methyl-β-phenyl-propiophenon,
2-[2'-Hydroxy-3'-(2-methoxyethylamino)-propoxy]-4-methyl-β-phenyl-propiophenon,
2-[2'-Hydroxy-3'-(4-hydroxybut-2-ylamino)-propoxy]-4-methyl-β-phenyl-propiophenon,
2-[2'-Hydroxy-3'-(4-hydroxy-4-phenyl-piperidino)-propoxy]-4-methyl-β-phenyl-propiophenon,
2-(2'-Hydroxy-3'-piperidino-propoxy)-4-methyl-β-phenyl-pro-piophenon,
2-(2'-Hydroxy-3'-n-propylamino-propoxy)-5-methoxy-β-phenyl-propiophenon,
2-(2'-Hydroxy-3'-n-butylamino-propoxy)-5-methoxy-β-phenyl-propiophenon,
2-(2'-Hydroxy-3'-isopropylamino-propoxy)-5-methoxy-β-phe-nyl-propiophenon,
2-(2'-Hydroxy-3'-tert.-butylamino-propoxy)-5-methoxy-β-phenyl-propiophenon,
2-[2'-Hydroxy-3'-(2-methoxyethylamino)-propoxy]-5-methoxy-β-phenyl-propiophenon,
2-[2'-Hydroxy-3'-(4-hydroxybut-2-ylamino)-propoxy]-5-meth-oxy-β-phenyl-propiophenon,
2-[2'-Hydroxy-3'-(4-hydroxy-4-phenyl-piperidino)-propoxy]-5-methoxy-β-phenyl-propiophenon,
2-(2'-Hydroxy-3'-piperidino-propoxy)-5-methoxy-β-phenyl-propiophenon,
2-(2'-Hydroxy-3'-n-propylamino-propoxy)-5-fluor-β-phenyl-propiophenon,
2-(2'-Hydroxy-3'-n-butylamino-propoxy)-5-fluor-β-phenyl-propiophenon,
2-(2'-Hydroxy-3'-iso-propylamino-propoxy)-5-fluor-β-phenyl-propiophenon,
2-(2'-Hydroxy-3'-tert.-butylamino-propoxy)-5-fluor-β-phenyl-propiophenon,
2-[2'-Hydroxy-3'-(2-methoxyethylamino)-propoxy]-5-fluor-β-phenyl-propiophenon,
2-[2'-Hydroxy-3'-(4-hydroxybut-2-ylamino)-propoxy]-5-fluor-β-phenyl-propiophenon,
2-[2'-Hydroxy-3'-(4-hydroxy-4-phenyl-piperidino)-propoxy]-5-fluor-β-phenyl-propiophenon,
2-(2'-Hydroxy-3'-piperidino-propoxy)-5-fluor-β-phenyl-propiophenon,
2-(2'-Hydroxy-3'-n-propylamino-propoxy)-4-fluor-β-phenyl-propiophenon,
2-(2'-Hydroxy-3'-n-butylamino-propoxy)-4-fluor-β-phenyl-propiophenon,
2-(2'-Hydroxy-3'-isopropylamino-propoxy)-4-fluor-β-phenyl-propiophenon,
2-(2'-Hydroxy-3'-tert.-butylamino-propoxy)-4-fluor-β-phenyl-propiophenon, .
2-[2'-Hydroxy-3'-(2-methoxyethylamino)-propoxy]-4-fluor-β-phenyl-propiophenon,
2-[2'-Hydroxy-3'-(4-hydroxybut-2-ylamino)-propoxy]-4-fluor-β-phenyl-propiophenon,
2-[2'-Hydroxy-3'-(4-hydroxy-4-phenyl-piperidino)-propoxy]-4-fluor-β-phenyl-propiophenon,
2-(2'-Hydroxy-3'-piperidino-propoxy)-4-fluor-β-phenyl-pro-piophenon,
2-(2'-Hydroxy-3'-n-propylamino-propoxy)-4-chlor-β-phenyl-propiophenon,
2-(2'-Hydroxy-3'-n-butylamino-propoxy)-4-chlor-β-phenyl-propiophenon,
2-(2'-Hydroxy-3'-isopropylamino-propoxy)-4-chlor-β-phenyl-propiophenon,
2-(2'-Hydroxy-3'-tert.-butylamino-propoxy)-4-chlor-β-phenyl-propiophenon,
2-[2'-Hydroxy-3'-(2-methoxyethylamino)-propoxy]-4-chlor-β-phenyl-propiophenon,
2-[2'-Hydroxy-3'-(4-hydroxybut-2-ylamino)-propoxy]-4-chlor-β-phenyl-propiophenon,
2-[2'-Hydroxy-3'-(4-hydroxy-4-phenyl-piperidino)-propoxy]-4-chlor-β-phenyl-propiophenon,
2-(2'-Hydroxy-3'-piperidino-propoxy)-4-chlor-β-phenyl-pro-piophenon,
2-(2'-Hydroxy-3'-n-propylamino-propoxy)-5-n-propoxy-β-phenyl-propiophenon,
2-(2'-Hydroxy-3'-n-butylamino-propoxy)-5-n-propoxy-β-phenyl-propiophenon,
2-(2'-Hydroxy-3'-isopropylamino-propoxy)-5-n-propoxy-β-phenyl-propiophenon,
2-(2'-Hydroxy-3'-tert.-butylamino)-propoxy)-5-n-propoxy-β-phenyl-propiophenon,
2-[2'-Hydroxy-3'-(2-methoxyethylamino)-propoxy-5-n-propoxy-β-phenyl-propiophenon,
2-[2'-Hydroxy-3'-(4-hydroxybut-2-ylamino)-propoxy]-5-n-pro-oxy-β-phenyl-propiophenon,
2-[2'-Hydroxy-3'-(4-hydroxy-4-phenyl-piperidino)-propoxy]-5-n-propoxy-β-phenyl-propiophenon,
2-(2'-Hydroxy-3'-piperidino-propoxy)-5-n-propoxy-β-phenyl-propiophenon,
2-(2'-Hydroxy-3'-n-propylamino-propoxy)-5-n-propyl-β-phenyl-propiophenon,
2-(2'-Hydroxy-3'-n-butylamino-propoxy)-5-n-propyl-β-phenyl-propiophenon,
2-(2'-Hydroxy-3'-isopropylamino-propoxy)-5-n-propyl-β-phenyl-propiophenon,
2-(2'-Hydroxy-3'-tert.-butylamino-propoxy)-5-n-propyl-β-phenyl-propiophenon,
2-[2'-Hydroxy-3'-(2-methoxyethylamino)-propoxy]-5-n-propyl-β-phenyl-propiophenon,
2-[2'-Hydroxy-3'-(4-hydroxybut-2-ylamino)-propoxy]-5-n-propyl-β-phenyl-propiophenon,
2-[2'-Hydroxy-3'-(4-hydroxy-4-phenyl-piperidino)-propoxy]-5-n-propyl-β-phenyl-propiophenon,
2-(2'-Hydroxy-3'-piperidino-propoxy)-5-n-propyl-β-phenyl-propiophenon,

2-(2'-Hydroxy-2'-n-propylamino-propoxy)-4-n-butyl-β-phenyl-propiophenon,
2-(2'-Hydroxy-3'-n-butylamino-propoxy)-4-n-butyl-β-phenyl-propiophenon,
2-(2'-Hydroxy-3'-isopropylamino-propoxy)-4-n-butyl-β-phenyl-propiophenon,
2-(2'-Hydroxy-3'-tert.-butylamino-propoxy)-4-n-butyl-β-phenyl-propiophenon,
2-[2'-Hydroxy-3'-(2-methoxyethylamino)-propoxy]-4-n-butyl-β-phenyl-propiophenon,
2-[2'-Hydroxy-3'-(4-hydroxybut-2-ylamino)-propoxy]-4-n-butyl-β-phenyl-propiophenon,
2-[2'-Hydroxy-3'-(4-hydroxy-4-phenyl-piperidino)-propoxy]-4-n-butyl-β-phenyl-propiophenon,
2-(2'-Hydroxy-3'-piperidino-propoxy)-4-n-butyl-β-phenyl-propiophenon,
2-(2'-Hydroxy-3'-n-butylamino-propoxy)-4-hydroxy-β-phenyl-propiophenon,
2-(2'-Hydroxy-3'-isopropylamino-propoxy)-4-hydroxy-β-phenyl-propiophenon,
2-(2'-Hydroxy-3'-tert.-butylamino-propoxy)-4-hydroxy-β-phenyl-propiophenon,
2-[2'-Hydroxy-3'-(2-methoxyethylamino)-propoxy]-4-hydroxy-β-phenyl-propiophenon,
2-[2'-Hydroxy-3'-(4-hydroxybut-2-ylamino)-propoxy]-4-hydroxy-β-phenyl-propiophenon,
2-[2'-Hydroxy-3'-(4-hydroxy-4-phenyl-piperidino)-propoxy]-4-hydroxy-β-phenyl-propiophenon,
2-(2'-Hydroxy-3'-piperidino-propoxy)-4-hydroxy-β-phenyl-propiophenon,
2-(2'-Hydroxy-3'-n-butylamino-propoxy)-5-hydroxy-β-phenyl-propiophenon,
2-(2'-Hydroxy-3'-isopropylamino-propoxy)-5-hydroxy-β-phenyl-propiophenon,
2-(2'-Hydroxy-2'-tert.-butylamino-propoxy)-5-hydroxy-β-phenyl-propiophenon,
2-[2'-Hydroxy-3'-(2-methoxyethylamino)-propoxy]5-hydroxy-β-phenyl-propiophenon,
2-[2'-Hydroxy-3'-(4-hydroxybut-2-ylamino)-propoxy]-5-hy-droxy-β-phenyl-propiophenon,
2-[2'-Hydroxy-3'-(4-hydroxy-4-phenyl-piperidino)-propoxy]-5-hydroxy-β-phenyl-propiophenon,
2-[2'-Hydroxy-3'-piperidino-propoxy)-5-hydroxy-β-phenyl-propiophenon,
2-(2'-Hydroxy-3'-n-propylamino-propoxy)-β-(2-methyl-phenyl)-propiophenon,
2-(2'-Hydroxy-3'-n-butylamino-propoxy)-β-(2-methyl-phenyl)-propiophenon,
2-(2'-Hydroxy-3'-isopropylamino-propoxy)-β-(2-methyl-phe-nyl)-propiophenon,
2-(2'-Hydroxy-3'-tert.-butylamino-propoxy)-β-(2-methyl-phenyl)-propiophenon,
2-[2'-Hydroxy-3'-(2-methoxyethylamino)-propoxy]-β-(2-methylphenyl)-propiophenon,
2-[2'-Hydroxy-3'-(4-hydroxy-but-2-ylamino)-propoxy]-β-(2-methylphenyl)-propiophenon,
2-[2'-Hydroxy-3'-(4-hydroxy-4-phenyl-piperidino)-propoxy]-β-(2-methylphenyl)-propiophenon,
2-(2'-Hydroxy-3'-piperidino-propoxy)-β-(2-methylphenyl)-propiophenon,
2-(2'-Hydroxy-3'-n-propylamino-propoxy)-β-(3-methoxyphenyl)-propiophenon,
2-(2'-Hydroxy-3'-n-butylamino-propoxy)-β-(3-methoxyphenyl)-propiophenon,
2-(2'-Hydroxy-3'-isopropylamino-propoxy)-β-(3-methoxy-phenyl)-propiophenon
2-(2'-Hydroxy-3'-tert.-butylamino-propoxy)-β-(3-methoxy-phenyl)-propiophenon,
2-[2'-Hydroxy-3'-(2-methoxyethylamino)-propoxy]-β-(3-meth-oxyphenyl)-propiophenon,
2-[2'-Hydroxy-3'-(4-hydroxybut-2-ylamino)-propoxy]-ß -(3-methoxyphenyl)-propiophenon,
2-[2'-Hydroxy-3'-(4-hydroxy-4-phenyl-piperidino)-propoxy]-β-(3-methoxyphenyl)-propiophenon,
2-(2'-Hydroxy-3'-piperidino-propoxy)-β-(3-methoxyphenyl)-propiophenon,
2-(2'-Hydroxy-3'-n-propylamino-propoxy)-β-(3,4-methylen-dioxyphenyl)-propiophenon,
2-(2'-Hydroxy-3'-n-butylamino-propoxy)-β-(3,4-methylendioxy-phenyl)-propiophenon,
2-(2'-Hydroxy-3'-isopropylamino-propoxy)-β-(3,4-methylen-dioxyphenyl)-propiophenon,
2-(2'-Hydroxy-3'-tert.-butylamino-propoxy)-β-(3,4-methylen-dioxyphenyl)-propiophenon,
2-[2'-Hydroxy-3'-(2-methoxyethylamino)-propoxy]-β-(3,4-methylendioxyphenyl)-propiophenon,
2-[2'-Hydroxy-3'-(4-hydroxybut-2-ylamino)-propoxy]-β-(3,4-methylendioxyphenyl)-propiophenon,
2-[2'-Hydroxy-3'-(4-hydroxy-4-phenyl-piperidino)-propoxy]-β-(3,4-methylendioxyphenyl)-propiophenon,
2-(2'-Hydroxy-3'-piperidino-propoxy)-β-(3,4-methylendioxy-phenyl)-propiophenon,
2-(2'-Hydroxy-3'-n-propylamino-propoxy)-β-(3,4-dichlor-phenyl)-propiophenon,
2-(2'-Hydroxy-3'-n-butylamino-propoxy)-β-(3,4-dichlorphenyl)-propiophenon,
2-(2'-Hydroxy-3'-isopropylamino-propoxy)-β-(3,4-dichlor-phenyl)-propiophenon,
2-(2'-Hydroxy-3'-tert.-butylamino-propoxy)-β-(3,4-dichlor-phenyl)-propiophenon,
2-[2'-Hydroxy-3'-(2-methoxyethylamino)-propoxy]-β-(3,4-dichlorphenyl)-propiophenon,
2-[2-Hydroxy-3'-(4-hydroxybut-2-ylamino)-propoxy]-β-(3,4-dichlorphenyl)-propiophenon,
2-[2':Hydroxy-3'-(4-hydroxy-4-phenyl-piperidino)-propoxy]-β-(3,4-dichlorphenyl)-propiophenon,
2-(2-Hydroxy-3'-piperidino-propoxy)-β-(3,4-dichlorphenyl)-propiophenon,
2-(2'-Hydroxy-3'-n-propylamino-propoxy)-β-(3-hydroxyphe-nyl)-propiophenon,
2-(2'-Hydroxy-3'-n-butylamino-propoxy)-β-(3-hydroxyphenyl)-propiophenon,
2-(2'-Hydroxy-3'-isopropylamino-propoxy)-β-(3-hydroxyphe-nyl)-propiophenon,
2-(2'-Hydroxy-3'-tert.-butylamino-propoxy)-β-(3-hydroxy-phenyl)-propiophenon,
2-[2'-Hydroxy-3'-(2-methoxyethylamino)-propoxy]-β-(3-hy-droxyphenyl)-propiophenon,
2-[2'-Hydroxy-3'-(4-hydroxy-but-2-ylamino)-propoxy]-β-(3-hydroxyphenyl)-propiophenon,
2-[2'-Hydroxy-3'-(4-hydroxy-4-phenyl-piperidino)-propoxy]-β-(3-hydroxyphenyl)-propiophenon,
2-(2'-Hydroxy-3'-piperidino-propoxy)-β-(3-hydroxyphenyl)-propiophenon,
2-(2'-Hydroxy-3'-n-propylamino-propoxy)-β-(4-hydroxyphe-nyl)-propiophenon,
2-(2'-Hydroxy-3'-n-butylamino-propoxy)-β-(4-hydroxyphenyl)-propiophenon,
2-(2'-Hydroxy-3'-isopropylamino-propoxy)-β-(4-hydroxyphe-nyl)-propiophenon,

2-(2'-Hydroxy-3'-tert.-butylamino-propoxy)-β-(4-hydroxy-phenyl)-propiophenon,
2-[2'-Hydroxy-3'-(2-methoxyethylamino)-propoxy]-β-(4-hy-droxyphenyl)-propiophenon,
2-[2'-Hydroxy-3'-(4-hydroxybut-2-ylamino)-propoxy]-β-(4-hydroxyphenyl)-propiophenon,
2-[2'-Hydroxy-3'-(4-hydroxy-4-phenyl-piperidino)-propoxy]-β-(4-hydroxyphenyl)-propiophenon,
2-(2'-Hydroxy-3'-piperidino-propoxy)-β-(4-hydroxyphenyl)-propiophenon,
2-(2'-Hydroxy-3'-n-propylamino-propoxy)-β-(2-dimethyl-aminophenyl)-propiophenon,
2-(2'-Hydroxy-3'-piperidino-propoxy)-β-(2-dimethylamino-phenyl)-propiophenon,
2-(2'-Hydroxy-3'-n-propylamino-propoxy)-β-(3-dimethyl-aminophenyl)-propiophenon,
2-[2'-Hydroxy-3'-(N,N-diisopropylamino)-propoxy]-β-(3-diethylaminophenyl)-propiophenon,
2-(2'-Hydroxy-3'-morpholino-propoxy)-β-(2-piperidino-phenyl)-propiophenon,
2-(2'-Hydroxy-3'-n-propylamino-propoxy)-β-(4-piperidino-phenyl)-propiophenon,
2-(2'-Hydroxy-3'-n-propylamino-propoxy)-β-(4-diisopropyl-aminophenyl)-propiophenon,
2-(2'-Hydroxy-3'-n-propylamino-propoxy)-β-(4-piperidino-phenyl)-propiophenon,
2-(2'-Hydroxy-3'-n-propylamino-propoxy)-5-hydroxy-β-(4-dimethylaminophenyl)-propiophenon,
2-(2'-Hydroxy-3'-n-propylamino-propoxy)-5-mathoxy-β-(4-diethylaminophenyl)-propiophenon,
2-(2'-Hydroxy-3'-piperidino-propoxy)-5-methoxy-β-(4-pipe-ridinophenyl)-propiophenon.

Die erfindungsgemäßen Verbindungen können hergestellt werden, indem man

a) eine Verbindung der Formel II

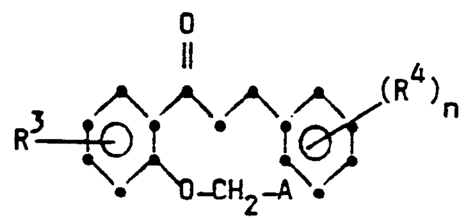

II

in der A den Rest 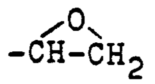 oder 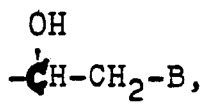,

wobei B für eine nucleofuge Abgangsgruppe steht, bedeutet und $R^3$, $R^4$ und n dia angegebenen Bedeutungen haben, mit einem Amin der Formel III

$HNR^1R^2$ III,

in der $R^1$ und $R^2$ die angegabenen Bedeutungen haben, umsetzt oder

b) eine Verbindung der Formel IV

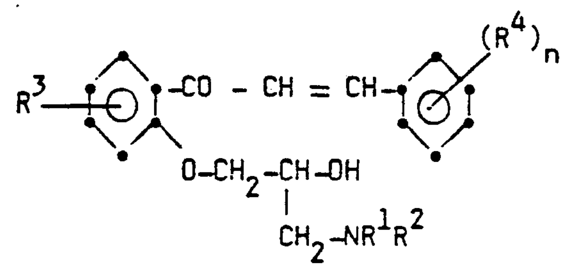

IV

worin $R^1$, $R^2$, $R^3$, $R^4$ und n die angegebene Bedeutung besitzen, katalytisch hydriert, und gegebenenfalls die so erhaltenen Verbindungen in ihre Säureadditions-salze physiologisch verträglicher Säuren überführt.

Im Falle des Verfahrens a) stellt die Abgangsgruppe B bevorzugt ein Halogenatom, insbesondere ein Chlor-, Brom-oder Iodatom dar. Weiterhin kommen beispialsweise als nucleofuge Abgangsgruppen aromatische oder aliphatische Sulfonsäurereste in Betracht, wie der p-Toluolsulfonsäure-oder der Methänsulfonsäurerest.

Die Umsetzungen werden bei Raumtamperaturen oder bei höheren Temperaturen, zweckmäßig bei Temperaturen von 50 bis 120°C, durchgeführt. Die Umsetzungen können unter Atmosphärendruck oder in einem geschlossenen Gefäß unter erhöhtem Druck gegebenenfalls unter Erwärmung auf den angegebenen Temperaturbereich durchgeführt werden.

Die Ausgangsverbindungen können direkt, d.h. ohne Zugabe eines Verdünnungs- oder Lösungsmittels, umgesetzt werden. Zweckmäßigerweise werden die Umsetzungen jedoch in Gegen-wart eines inerten Verdünnungs- oder Lösungsmittels, bei-spielsweise eines niederen Alkohols mit 1 bis 4 C-Atomen, wie Methanol, Ethanol oder Propanol, vorzugsweise Isopro-panol oder Ethanol, eines niederen gesättigten Dialkylethers, Dialkylglykolethers oder cyclischen Ethers, wie Diethylethar, 1,2-Dimethoxyethan, Tetrahydrofuran oder Dioxan, Benzol oder eines Alkylbenzols, wie Toluol oder Xylol, oder eines aliphatischan Kohlenwasserstoffs, wie Hexan, Heptan oder Octan, eines niederen aliphatischen Ketons, wie Aceton, Methlethylketon oder Methylisobutyl-keton, eines Dialkylformamids, wie Dimethyl- oder Diethyl-formamid, von Dimethylsulfoxid oder in Gegenwart von Was-ser oder in Mischungen der genannten Lösungsmittel, durch-geführt. Auch ist das in

Überschüssiger Menge verwende-te Amin der allgemeinen Formel $HNR^1R^2$ gegebenenfalls als Verdünnungs- oder Lösungsmittel geeignet.

Bevorzugte Lösungsmittel bei der Umsetzung der Verbindun-gen II (R =

FIG07/10

mit einem Amin $HNR^1R^2$ sind niedere Alkohole, insbesondere Ethanol oder Isopropanol, wobei die Umsetzung bevorzugt bei Temperaturen von 50 bis 120° C und bei Normaldruck durchgeführt wird.

Die vollständige Umsetzung hängt von der Reaktionstem-peratur ab und ist im allgemeinen innerhalb von 2 bis 15 Stunden beendet. Das Reaktionsprodukt kann in an sich üblicher Weise gewonnen werden, z.B. durch Filtration oder Abdestillieren des Verdünnungs- oder Lösungsmittels aus dem Reaktionsgemisch. Eine Reinigung der erhaltenen Ver-bindung erfolgt in üblicher Weise, beispielsweise durch Umkristallisieren aus einem Lösungsmittel, Überführen in eine Säureadditionsverbindung oder durch Säulenchromato-graphie.

Die Ausgangsverbindungen der allgemeinen Formel II sind zum Teil bekannt oder können wie folgt hergestellt werden:

Ein entsprechend substituiertes Acetophenon wird mit einem Benzaldehyd zu den α,β-ungesättigten Ketonen nach litera-turbekannten Methoden kondensiert, wie sie beispielsweise in Org. Reactions Bad. 16, S. Iff, John Wiley Verlag, New York, 1968, in Houben-Weyl, Methoden der organischen Chemie, Bd. 7/2b, S 1457 ff. G. Thieme Verlag, Stuttgart, 1976 oder in Chem. Ber. 94, 26 (1961) beschrieben sind. Diese Ketone werden zu den entsprechenden 2-Hydroxy-β-phenyl-propiophenonen ebenfalls nach literaturbekannten Methoden katalytisch hydriert, wie es beispielsweise in R.N. Rylander, 'Catalytic Hydrogenation over Pt-Metals', S. 282, Academic Press 1967, beschrieben ist.

Die Überführung dieser 2-Hydroxy-β-phenyl-propiophenone in die Propiophenone der Formel II erfolgt durch Alkylierung mit einem Epihalogenhydrin oder einem 1,3-Dihalogen-2-pro-panol nach an sich bekannten Verfahren.

Als Epihalogenhydrine kommen Epichlorhydrin, Epibromhydrin und Epiiodhydrin und als 1,3-Dihalogen-2-propanole kommen insbesondere 1,3-Dichlor-2-propanol und 1,3-Dibrom-2-pro-panol in Betracht.

Die Umsetzungen der 2-Hydroxy-β-phenyl-propiophenone zur Herstellung der Verbindungen der Formel II werden bei Temperaturen von 50 bis 80° C und unter Normaldruck oder unter erhöhten Drucken in einem inerten Verdünnungs- oder Lösungsmittel, z.B. wie Aceton, Methanol, oder Dimethyl-formamid in Gegenwart einer Base wie Kaliumcarbonat als säurebindendes Mittel vorgenommen.

Die 2-Hydroxy-β-phenyl-propiophenone und die Ausgangs-verbindungen II können zum Teil ohne vorherige Reinigung direkt in die nachfolgenden Reaktionsschritte eingesetzt werden.

Das Verfahren b) gelingt gut in alkoholischer Lösung. Als Katalysatoren eigenen sich insbesondere Edelmetallkataly-satoren, wie Palladium, auf Kohle.

Die erfindungsgemäßen Verbindungen der Formel I besitzen am Kohlenstoffatom 2 der aliphatischen Seitenkette ein Chiralitätszentrum und werden als Racemate erhalten, die durch bekannte Methoden, beispielsweise durch Bildung diastereomerer Salze mit optisch aktiven Hilfssäuren, wie Dibenzoylweinsäure, Campher-10-sulfonsäure, Ditoluylwein-säure oder 3-Brom-campher-8-sulfonsäure, in die optisch aktiven Antipoden getrennt werden können.

Gegebenenfalls werden die erhaltenen erfindungsgemäßen Verbindungen in das Säureadditionssalz einer physiologisch verträglichen Säure überführt. Als übliche physiologisch verträgliche organische oder anorganische Säuren kommen beispielsweise in Betracht: Salzsäure, Bromwasserstoff-säure, Phosphorsäure oder Schwefelsäure, und als organische Säuren beispielsweise Oxalsäure, Maleinsäure, Fumarsäure, Milchsäure, Weinsäure, Äpfelsäure, Zitronensäure, Salicyl-säure, Adipinsäure oder Benzoesäure oder können aus Fort-schritte der Arzneimittelforschung Band 10, Seiten 224 bis 225, Birkhäuser Verlag, Basel und Stuttgart, 1966 entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Ethanol oder Propanol, oder einem niederen Keton, wie Aceton, Methylethylketon oder Methylisobutylketon, oder einem Ether, wie Diethyl-ether, Tetrahydrofuran oder Dioxan, erhalten. Zur besseren Kristallabscheidung können auch Mischungen der genannten Lösungsmittel verwendet werden. Darüber hinaus können pharmazeutisch vertretbare wäßrige Lösungen von Säure-Additionsverbindungen der Aminopropanolderivate der Formel I durch Auflösen der freien Basen in einer wäßrigen Säure-lösung hergestellt werden.

Die erfindungsgemäßen Verbindungen und ihre physiologisch verträglichen Säureadditionssalze sind wegen ihrer anti-arrhythmischen, β-sympatholytischen und Ca-antagonisti-schen Eigenschaften insbesondere zur Pharmakotherapie von Herzrhythmusstörungen und zur Prophylaxe des plötzlichen Herztodes sowie zur Behandlung der coronaren Herzkrankheit geeignet.

Die antiarrhythmische Wirksamkeit der erfindungsgemäßen Verbindungen wurde an isolierten linken Vorhöfen von männlichen Meerschweinchen (Stamm: Pirbright white, Gewicht 350 bis 450 g) bestimmt.

In einem Organbad (Volumen 125 ml) mit carbogengesättigter Tyrode (pH 7,4, Temperatur 32° C) suspendierte Vorhöfe waren mit 1,0 g vorbelastet und durch Rechteckimpulse von 1 Hz Grundrhythmus und doppelten Reizschwellenwerten (Rheobase: 0,2 bis 1,4 V, Chronaxie: 0,3 bis 0,5 msec) angetrieben.

Als Kriterium für antiarrhythmische Wirksamkeit wurde durch automatische kontinuierliche Frequenzerhöhung die Frequenz (Hz) bestimmt, bis zu der auf jeden Reiz eine Kontraktion folgt (maximale Folgefrequenz, MFF). Aus

der linearen Beziehung zwischen log Konzentration (mg/l) und der relativen Abnahme der maximalen Folgefrequenz ( Δ %) wurde die Konzentration berechnet, welche eine Abnahme der maximalen Folgefrequenz um 50 % bewirkt (EC 50 %).

Darüber hinaus wurden die neuen Verbindungen zur Bestim-mung der antiarrhythmischen Wirksamkeit männlichen Ratten (Stamm: Spraque Dawley, Gewicht 200 bis 230 g) oral oder i.v. appliziert. 45 min später wurden die Tiere mit Thiobutabartital-Natrium (100 mg/kg i.p.) narkotisiert. Als arrhythmogene Substanz diente Aconitin, das 60 min nach Substanzapplikation i.v. infundiert wurde (Dosierungs-geschwindigkeit: 0,005 mg/kg x min). Bei unbehandelten Tieren (n = 52) traten nach 2,74 ± 0,07 min im EKG Arrhythmien auf, deren Eintritt durch Antiarrhythmika dosisabhängig verzögert werden kann.

Aus der linearen Beziehung zwischen log Dosis (mg/kg) der Prüfsubstanzen und der relativen Verlängerung der Aconitininfusionsdauer (Δ %) wurde die Dosis bestimmt, welche die Infusionsdauer um 50 % verlängert (ED 50 %).

Als Vergleichssubstanz diente das bekannte Antiarrhythmi-kum Propafenon [2-(-2-Hydroxy-3-propylamino-propoxy)-3-phenyl-propiophenon-hydrochlorid].

Am isolierten Meerschweinchenvorhof (Tabelle 1) bewirken die erfindungsgemäßen Verbindungen eine Abnahme der MFF, welche die Wirksamkeit von Propafenon bis 3fach über-trifft. Auch am Modell der Aconitinarrhythmie der Ratte zeigt sich im Vergleich zu Propafenon eine starke bis sehr starke antiarrhythmische Wirkung (Tabelle 2). Die Verträg-lichkeit der erfindungsgemäßen Verbindungen in diesem Test ist gut. Daraus ergibt sich ein großer Abstand zwischen antiarrhythmisch wirksamer und toxischer Dosis.

**Tabelle 1:** Antiarrhythmische Wirkung am isolierten Meerschweinchenvorhof

| Substanz des Beispiels Nr. | Maximale Folgefrequenz EC 50 % mg/l 1) |
|---|---|
| 1 | 1,90 |
| 2 | 0,874 |
| 3 | 1,51 |
| 6 | 1,71 |
| 7 | 1,66 |
| 9 | 0,757 |
| 10 | 1,13 |
| 12 | 1,25 |
| 14 | 2,17 |
| 15 | 0,728 |
| 18 | 1,30 |
| 22 | 1,21 |
| 23 | 1,64 |
| 24 | 2,65 |
| 25 | 1,01 |
| 29 | 1,71 |
| 33 | 2,05 |
| 34 | 1,72 |
| 35 | 1,65 |

Forts. Tabelle 1

| Substanz des Beispiels Nr. | Maximale Folgefrequenz EC 50 % mg/l 1) |
|---|---|
| 46 | 0,778 |
| 47 | 1,45 |
| 48 | 0,923 |
| 49 | 1,82 |
| 55 | 0,854 |
| 57 | 2,04 |
| 58 | 1,92 |
| 60 | 1,82 |
| 64 | 1,36 |
| 67 | 0,658 |
| Propafenon | 2,02 |

1) Konzentration (mg/l, welche eine 50 %ige Abnahme der maximalen Folgefrequenz bewirkt

**Tabelle 2**

Antiarrhythmische Wirkung an der Aconitinarrhythmie der Ratte (Appl. per os)

| Substanz des | Beispiels Nr. | EC 50 % mg/kg 1) |
|---|---|---|
| | 1 | 15 |
| | 2 | 46,4 |
| | 3 | 38,2 |
| | 4 | 46,4 |
| | 8 | 21 |
| | 9 | 27 |
| | 10 | 46,4 |
| | 16 | 29 |
| | 23 | 31,6 |
| | 24 | 25,4 |
| | 25 | 11,2 |
| | 33 | 24,5 |
| | 34 | 21,5 |
| | 35 | 1,99 |
| | 36 | 6,92 |
| | 37 | 46,4 |
| | 38 | 21,5 |
| | 40 | 46,4 |
| | 49 | 31,6 |
| | 55 | 5,70 |
| | 56 | 3,40 |
| | 57 | 1,78 |
| | 58 | 2,33 |
| | 59 | 21,5 |
| | 60 | 3,25 |
| | 61 | 1,90 |
| | 62 | 21,5 |
| | 63 | 3,03 |
| | 66 | 21,5 |
| | 67 | 2,15 |
| | 68 | 46,4 |
| | 69 | 31,6 |
| | 71 | 0,510 2) |
| Propafenon | | 58,7 |
| | | 0,724 2) |

1) Dosis (mg/kg), die eine 50 %ige Verlängerung der Aconitininfusionsdauer bewirkt
2) Applikation i.v.

Die neuen Verbindungen können in üblicher Weise oral oder intravenös verabfolgt werden. Die Dosierung hängt vom Alter, Zustand und Gewicht des Patienten sowie von der Applikationsart ab. In der Regel beträgt die tägliche Wirkstoffdosis zwischen etwa 5 und 75 mg/kg Körpergewicht bei oraler Gabe und zwischen etwa 1 und 10 mg/kg Körper-gewicht bei parenteraler Gabe.

Die neuen Verbindungen können in den gebräuchlichen gale-nischen Applikationsformen fest oder flüssig angewendet werden, z.B. als Tabletten, Filmtabletten, Kapseln, Pulver, Granulate, Dragees, Suppositorien oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weich-machern, Netzmitteln, Dispergiermitteln, Emulgatoren, Lösungsmitteln, Retardierungsmitteln und/oder Antioxidan-tien verarbeitet werden (vgl. H. Sucker et al: Pharmazeu-tische Technologie, Thieme-Verlag, Stuttgart, 1978). Die so erhaltenen Applikationsformen enthalten den Wirkstoff normalerweise in einer Menge von 1 bis 99 Gewichtsprozent.

Die folgenden Beispiele sollen die Erfindung näher erläu-tern.

A) Herstellung der Ausgangsverbindungen.

**Beispiel I**

3-Oxo-1-phenyl-3-(2'-hydroxy-5'-chlor-phenyl)-propen

Zu einer Mischung aus 180 ml $H_2O$ und 37 g 50 %iger Natron-lauge wurden bei Raumtemperatur 34,1 g 5-Chlor-2-hydroxy-acetophenon und 22 g Benzaldehyd gegeben. Die Reaktions-mischung wurde auf 55-60° C unter gutem Rühren aufgeheizt und ca. 1 h bei dieser Temperatur gerührt. Unter guter Kühlung wurde anschließend mit 25 %iger $H_2SO_4$ (ca. 100 ml) schwach angesäuert; der Niederschlag wurde abgesaugt, mit $H_20$ gut nachgewaschen und aus Methanol/Aceton (9 : 1) umkristallisiert. Es wurden 38,7 g 3-Oxo-1-phenyl-3-(2'-hydroxy-5'-chlorphenyl)-propen vom Fp. 88-90° C isoliert.

Analog wurden folgende Verbindungen hergestellt:

3-Oxo-1-(4'methoxyphenyl)-3-(2'-hydroxyphenyl)-propen, Fp: 91-92° C;
3-Oxo-1-(2',4'-dichlorphenyl)-3-(2'-hydroxyphenyl)-pro-pen, Fp: 169-172° C;
3-Oxo-1-(2'-methoxyphenyl)-3-(2'-hydroxyphenyl)-propen, Fp: 110-112° C;
3-Oxo-1-(3',4'-dimethoxyphenyl)-3-(2'-hydroxyphenyl)-propen, Fp: 11-116° C;
3-Oxo-1-(3',4',5'-trimethoxyphenyl)-3-(2'-hydroxyphenyl)-propen, Fp: 152-153° C;
3-Oxo-1-(4'-dimethylaminophenyl)-3-(2'-hydroxyphenyl)-propen, Fp: 174-176° C;
3-Oxo-1-(4'-chlorphenyl)-3-(2'-hydroxyphenyl)-propen, Fp: 149-150° C;
3-Oxo-1-(4'-methylphenyl)-3-(2'-hydroxyphenyl)-propen, Fp: 118-120° C;
3-Oxo-1-(4'-fluorphenyl)-3-(2'-hydroxyphenyl)-propen, Fp: 115-119° C;
3-Oxo-1-(3',4'-dimethoxyphenyl)-3-(2'-hydroxy-5'-methyl-phenyl)-propen, Fp: 142-145° C;
3-Oxo-1-(4'-chlorphenyl)-3-(2'-hydroxy-5'-methylphenyl)-propen, Fp: 151-156° C;
3-Oxo-1-(4'-methoxyphenyl)-3-(2'-hydroxy-5'-chlorphenyl)-propen, Fp: 108-111° C;
3-Oxo-1-(4'-fluorphenyl)-3-(2'-hydroxy-5'-chlorphenyl)-propen, Fp: 183-185° C;
3-Oxo-1-(4'-diethylaminophenyl)-3-(2'-hydroxyphenyl)-pro-pen, Fp: 117-119° C;
3-Oxo-1-(4'-dimethylaminophenyl)-3-(2'-hydroxy-5'-chlor-phenyl)-propen, Fp: 159-162° C;
3-Oxo-1-(4'-dimethylaminophenyl)-3-(2'-hydroxy-5'-methyl-phenyl)-propen, Fp: 125-129° C.

**Beispiel II**

a) 2-Hydroxy-5-chlor-β-phenyl-propiophenon

25 g 3-Oxo-1-phenyl-3-(2'-hydroxy-5'-chlorphenyl)-propen (0,097 mol) wurden in 200 ml Tetrahydrofuran gelöst und in Gegenwart von 1,5 g Katalysator (0,5 % Pd auf $Al_20_3$) bei Normaldruck bei einer Temperatur von 40

bis 50° C hydriert. Nach 2 bis 3 h war die Wasser-stoffaufnahme beendet (2100 ml). Anschließend wurde vom Katalysator abfiltriert und das Filtrat durch Destillation unter vermindertem Druck vom Lösungsmit-tel befreit. Der verbleibende Rückstand wurde aus Methanol umkristallisiert. Ausbeute: 19,9 g, Fp: 56-57° C.

Analog wurden folgende Verbindungen hergestellt:

2-Hydroxy-β-(4-methoxyphenyl)-propiophenon,Fp: 51-54° C 2-Hydroxy-β-(2,4-dichlorphenyl)-propiophenon, Fp: 106° C 2-Hydroxy-β-(4-dimethylaminophenyl)-propiophenon, Fp: 69-71° C

2-Hydroxy-β-(3,4-dimethoxyphenyl)-propiophenon, Fp: 84-87° C

2-Hydroxy-β-(4-dimethylaminophenyl)-propiophenon, Fp: 62° C 2-Hydroxy-β-(4-diethylaminophenyl)-propiophenon, Fp: 49-5loC

2-Hydroxy-5-methyl-β-(4-dimethylaminophenyl)-propiophenon, Fp: 90-92° C

2-Hydroxy-5-chlor-β-(4-dimethylaminophenyl)-propiophenon, Fp: 99° C

2-Hydroxy-β-(4-chlorphenyl)-propiophenon, $C_{15}H_{13}C1O_2$ (260,72); ölig

Ber.: C 69,10 H 5,03 Cl 13,60

Gef.: C 69,5 H 5,4 Cl 13,2

2-Hydroxy-β-(2-methoxyphenyl)-propiophenon $C_{16}H_{16}0_3$ (256,30); ölig

Ber.: C 74,98 H 6,29

Gef.: C 74,7 H 6,4

Die weiteren Propiophenone wurden ohne weitere Reinigung eingesetzt.

**Beispiel III**

2-Hydroxy-4-methoxy-β-phenyl-propiophenon

16,6 g (100 mmol) 2-Hydroxy-4-methoxy-acetophenon und 10,6 g (100 mmol) Benzaldehyd wurden in 300 ml Metha-nol gelöst und 8 g (200 mmol) Natriumhydroxid, das in 30 ml $H_20$ gelöst war, zugetropft. Die Lösung färbte sich gelb. Das Reaktionsprodukt wurde 72 h bei Raumtemperatur gerührt. Anschließend wurde mit 200 ml Methanol verdünnt und ca. 2 g Pd/C (5 %) zugesetzt. Die gelbe Lösung wurde hydriert. Die Wasserstoffaufnahme betrug 1800 ml. Die Suspension wurde abfiltriert und die Mutterlauge mit ca. 10 ml konz. Essigsäure auf pH 5 eingestellt. Es fielen Kristalle aus, die abgesaugt wurden. Ausbeute 18 g (= 70,2 %); Fp: 100° C.

**Beispiel IV**

2-(2',3'-Epoxypropoxy)-5-chlor-β-phenyl-propiophenon

69 g (0,265 mol) 2-Hydroxy-5-chlor-β-phenyl-propiophenon wurden mit 125,4 g (0,915 mol) Epibromhydrin, 70 ml Di-methylformamid und 48,7 g (0,353 mol) wasserfreiem $K_2CO_3$ 5 h bei 60° C gut gerührt. Nach dem Abkühlen wurde mit 250 ml $H_20$ versetzt und die organische Phase durch Destil-lation unter vermindertem Druck vom überschüssigen Epibrom-hydrin befreit. Der verbleibende Rückstand wurde aus Cyclohexan/Methyl-tert.-butylether umkristallisiert. Ausbeute: 67 g (= 79 %); Fp: 46-47° C.

Analog wurde hergestellt:

2-(2',3'-Epoxypropoxy)-5-chlor-β-(4-dimethylaminophenyl)-propiophenon, Fp: 93-95° C.

Analog wurden alle übrigen Ausgangsmaterialien herge-stellt, die jedoch ohne weitere Reinigung umgesetzt wurden.

**Beispiel V**

3-Oxo-1-phenyl-3-[(2'-hydroxy-3'-n-propylamino-propoxy)-4-benzyloxy-pheny1]-propen

a) 2-Hydroxy-4-benzyloxy-acetophenon

15,2 g (100 mmol) 2,4-Dihydroxy-acetophenon, 17,1 g (100 mmol) Benzylbromid und 13,8 g (100 mmol) Kaliumcarbonat wurden 8 h unter Rühren in 150 ml Aceton am Rückfluß erhitzt (Farbänderung von violett nach rotbraun). Nach dem Abkühlen wurde das Lösungsmittel abdestilliert. Der Rückstand wurde in 100 ml Essigsäureethylester aufgenommen und mit 100 ml 1 N Natron-lauge ausgeschüttelt. Die Phasen wurden getrennt. Die organische Phase wurde mit 110 ml 1 N HCl angesäuert und erneut abgetrennt. Die organische Phase wurde mit Natriumsulfat getrocknet. Das Trockenmittel wurde abfiltriert und das Lösungsmittel abdestilliert (Rohausbeute 20,5 g). Das Rohprodukt wurde aus 180 ml Ethanol umkristalliert. Ausbeute: 17.5 g: Fp. 108-109° C.

b) 3-Oxo-1-phenyl-3-(2'-hydroxy-4-benzyloxyphenyl)-propen

17,63 g (72,6 mmol) 2-hyfroxy-4-benzyloxy-acetophenon, 17,60 g (165,8 mmol) Benzaldehyd und 35,20 g 50 %ige Natronlauge wurden 3 Tage in 200 ml Ethanol gerührt. Während der Reaktionszeit fielen Kristalle aus. Der Kristallbrei wurde mit 100 ml 5 N Salzsäure angesäuert und 10 min nachgerürt. Die Kristalle wurden abgesaugt (40,0 g feuchte Kristalle). Das Rohprodukt wurde aus 800 ml Ethanol umkristallisiert. Ausbeute: 13,6 g;

Fp. 116-118° C.

c) 3-Oxo-1-phenyl-3-[2'-2(2',3'-epoxypropoxy)-4'-benzyloxy-phenyl]-propen

13,55 g (41,0 mmol) 3-Oxo-1-phenyl-3-(2'-hydroxy-4-benzyloxy-phenyl)-propen wurden mit 1,64 g (41,0 mmol) Natriumhydroxid in 200 ml Epichlorhydrin 4 h unter Rüfluß erhitzt. Das entstehende Reaktionswasser wurde während des Siedens abgetrennt. Anschlie-ßend wurde das Reaktionsprodukt abgekühlt und das gebildete Natriumchlorid abgesaugt. Das Filtrat wurde im Vakuum eingeengt. Der Rückstand wurde ohne Reinigung in die nächste Stufe eingesetzt.

d) 3-Oxo-1-phenyl-3[(2'-hydroxy-3'-n-propylamino-propoxy)-4-benzyloxy-phenyl]-propen

16,3 g (42,2 mmol) 3-Oxo-1-phenyl-3-[2'-(2',3'-epoxy-propoxy)-4'-benzyloxy-phenyl]-propen wurden in 200 ml n-Propylamin 4 h am Rückfluß erhitzt. Das überschüs-sige Amin wurde anschließend abdestilliert. Das so erhaltene Rohprodukt wird ohne weitere Reinigung direkt für die Herstellung des erfindungsgemäßen Endproduktes verwendet.

Analog wurde hergestellt:

3-Oxo-1-phenyl-3[2'-hydroxy-3'-n-propylamino-propoxy)-5-benzyloxy-phenyl]-propen

B) Herstellung der erfindungsgemäßen Verbindungen:

**Beispiel 1**

2-[2'-Hydroxy-3'-(4-hydroxybut-2-yl-amino)-propoxy]-β-phenyl-propiophenon-hydrochlorid

5 g 2-(2',3'-Epoxypropoxy)-β-phenyl-propiophenon und 1,55 g 4-Hydroxy-but-2-yl-amin wurden in 100 ml Isopropa-nol gelöst und 8 h auf dem Wasserbad zum Rückfluß erhitzt. Nach dem Erhalten wurde das Lösungsmittel unter verminder-tem Druck abdestilliert; es verblieben 5,5 g öliger Rück-stand. Das Rohprodukt wurde über eine Kieselgelsäule gereinigt (Laufmittel: Toluol/Methanol 70 : 30) und die sauberen Fraktionen in das Hydrochlorid überführt. Aus-beute: 2,9 g (40,5 %); Fp: 88,5° C (Aceton/Ether).

**Beispiel 2**

2-[2'-Hydroxy-3'-(3-methoxyprop-2-ylamino)-propoxy]-β-phenyl-propiophenon-hydrochlorid

10 g 2-(2',3'-Epoxypropoxy)-β-phenyl-propiophenon (0,035 Mol) und 16 g 3-Methoxyprop-2-yl-amin (0,18 Mol) wurden in 150 ml Isopropanol gelöst und die Mischung 6 h auf dem Wasserbad unter Rückfluß gehalten. Anschließend wurde das Lösungsmittel unter vermindertem Druck abdestil-liert. Aus dem öligen Rückstand (12,85 g) wurde mit Hilfe von etherischer Salzsäure das Hydrochlorid hergestellt und aus Aceton/Ether umkristalliert. Ausbeute: 9,8 g (68 %); Fp: 119° C.

Analog wurden hergestellt:

3. 2-(2'-Hydroxy-3'-cyclopropylamino-propoxy)-β-phenyl-propiophenon-hydrochlorid, Fp: 153° C
4. 2-(2'-Hydroxy-3'-cyclohexylamino-propoxy)-β-phenyl-propiophenon-hydrochlorid, Fp: 162° C
5. 2-(2'-Hydroxy-3'-allylamino-propoxy)-β-phenyl-propiophenon-hydrochlorid, Fp: 153° C
6. 2-[2'-Hydroxy-3'-(1-butin-3-ylamino)-propoxy]-β-phenyl-propiophenon-hydrochlorid, Fp: 133° C
7. 2-[2'-Hydroxy-3'-(3-methyl-1-butin-3-ylamino)-propoxy]-β – phenyl-propiophenon-hydrochlorid, Fp: 131-132° C
8. 2-[2'-Hydroxy-3'-(3-hydroxy-3-methyl-butylamino)-propoxy]-β-phenyl-propiophenon-hydrochlorid, Fp: 145-146° C
9. 2-[2'-Hydroxy-3'-(2-methoxyethylamino)-propoxy]-β-phenyl-propiophenon-hydrochlorid, Fp: 112-113° C
10. 2-[2'-Hydroxy-3'-(3-thiomethoxyprop-2-ylamino)-propoxy]-β-phenyl-propiophenon-hydrochlorid, Fp: 119° C
11. 2-[2'-Hydroxy-3'-(2-phenylethylamino)-propoxy]-β-phenyl-propiophenon-hydrochlorid, Fp: 157-158° C
12. 2-[2'-Hydroxy-3'-(2-phenylpropylamino)-propoxy]-β-phenyl-propiophenon-hydrochlorid, Fp: 152° C
13. 2-[2'-Hydroxy-3'-(1-methylbenzylamino)-propoxy]-β-phenyl-propiophenon-hydrochlorid, Fp: 119-120° C
14. 2-[2'-Hydroxy-3'-(2-(3,4-dimethoxyphenyl)-ethyl-amino)-propoxy]-β-phenyl-propiophenon-hydro-chlorid, Fp: 129° C
15. 2-[2'-Hydroxy-3'-[N-methyl-N-(2-(3 4-dimethoxy-phenyl)-ethylamino)]-propoxy-β-phenyl-propiophenon -hydrochlorid, Fp: 121° C
16. 2-[2'-Hydroxy-3'-(2-dimethylaminoethylamino)-propoxy]-β-phenyl-propiophenon-bis-hydrochlorid, Fp: 136-137° C
17. 2-[2'-Hydroxy-3'-(3-dimethylaminopropylamino)-propoxy]-β-phenyl-propiophenon-bis-hydrochlorid, Fp. 150-151° C
18. 2-[2'-Hydroxy-3'-(N,N-dimethylamino)-propoxy]-β-phenyl-propiophenon-hydrochlorid, Fp: 154° C
19. 2-[2'-Hydroxy-3'-(N,N-di-isopropylamino)-propoxy]-β-phenyl-propiophenon-hydrochlorid, Fp: 131° C
20. 2-(2'-Hydroxy-3'-piperidino-propoxy)-β-phenyl-propio-phenon-hydrochlorid, Fp. 155-156° C
21. 2-(2'-Hydroxy-3'-morpholino-propoxy)-β-phenyl-propio-phenon-hydrochlorid, Fp: 153° C
22. 2-[2'-Hydroxy-3'-(3-phenyl-pyrrolidino)-propoxy]-β-phenyl-propiophenon-hydrochlorid, Fp: 128-130° C
23. 2-[2'-Hydroxy-3'-(4-hydroxypiperidino)-propoxy]-β-phenyl-propiophenon-hydrochlorid, Fp: 150-152° C
24. 2-[2'-Hydroxy-3'-(4-hydroxy-4-phenyl-piperidino)-propoxy]-β-phenyl-propiophenon-hydrochlorid, Fp. 107-108°C

25. 2-[2'-Hydroxy-3'-(4-methyl-1-piperazinyl)-propoxy]-β-phenyl-propiophenon-bis-hydrochlorid, Fp: 152-153°C
26. 2-{2'-Hydroxy-3'-[4-(2-methoxyphenyl)-1-piperazinyl]-propoxy}-β-phenyl-propiophenon, Fp: 119-120°C
27. 2-{2'-Hydroxy-3'-[4-(4-fluorphenyl)-1-piperazinyl]-propoxy{-β-phenyl-propiophenon, Fp: 93°C
28. 2-{2'-Hydroxy-3'-[2-(2-methylphenyl)-propylamino]-propoxy}-β-phenyl-propiophenon-hydrochlorid, Fp: 140-141°C
29. 2-(2'-Hydroxy-3'-n-propylamino-propoxy)-β-(2-methoxy-phenyl)-propiophenon-hydrochlorid, Fp: 123°C
30. 2-(2'-Hydroxy-3'-n-propylamino-propoxy)-β-(4-methoxy-phenyl)-propiophenon-hydrochlorid, Fp: 159°C
31. 2-(2'-Hydroxy-3'-n-propylamino-propoxy)-β-(4-chlor-phenyl)-propiophenon-hydrochlorid, Fp: 157°C
32. 2-(2'-Hydroxy-3'-n-propylamino-propoxy)-β-(2,4-di-chlorphenyl)-propiophenon-hydrochlorid, Fp: 119-123°C
33. 2-(2'-Hydroxy-3'-n-propylamino-propoxy)-β-(4-fluor-phenyl)-propiophenon-hydrochlorid, Fp: 144°C
34. 2-(2'-Hydroxy-3'-n-propylamino-propoxy)-β-(4-methyl-phenyl)-propiophenon-hydrochlorid, Fp: 148-149°C
35. 2-(2'-Hydroxy-3'-n-propylamino-propoxy)-β-(4-dimethyl-aminophenyl)-propiophenon-hydrochlorid, Fp: 165-166°C
36. 2-(2'-Hydroxy-3'-n-propylamino-propoxy)-β-(3,4-dimeth-oxyphenyl)-propiophenon-hydrochlorid, Fp: 185-186°C
37. 2-(2'-Hydroxy-3'-n-propylamino-propoxy)-5-chlor-β-phenyl-propiophenon-hydrochlorid, Fp: 163-164°C
38. 2-[2'-Hydroxy-3'-(3-methoxy-prop-2-yl-amino)-propoxy]-5-chlor-β-phenyl-propiophenon-hydrochlorid, Fp: 105-106°C
39. 2-(2'-Hydroxy-3'-cyclopropylamino-propoxy)-5-chlor-β-phenyl-propiophenon-hydrochlorid, Fp: 152°C
40. 2-(2'-Hydroxy-3'-piperidino-propoxy)-5-chlor-β-phenyl-propiophenon, Fp: 91-92°C
41. 2-[2'-Hydroxy-3'-(2-(3,4-dimethoxyphenyl)-ethylamino)-propoxy]-5-chlor-β-phenyl-propiophenon-hydrochlorid, Fp: 137-141°C
42. 2- 2'-Hydroxy-3'-[N-methyl-N-2-(3,4-dimethoxyphenyl)-ethylamino)]-propoxy -5-chlor-β-phenyl-propiophenon-hydrochlorid, Fp: 135°C
43. 2-[2'-Hydroxy-3'-(4-hydroxy-4-phenyl-piperidino)-prop-oxy]-5-chlor-β-phenyl-propiophenon-hydrochlorid, Fp: 214-216°C
44. 2-(2'-Hydroxy-3'-n-propylamino-propoxy)-5-chlor-β-(4-methoxy-phenyl)-propiophenon-hydrochlorid, Fp: 165-167°C
45. 2-(2'-Hydroxy-3'-n-propylamino-propoxy)-5-methyl-β-(4-chlorphenyl)-propiophenon-hydrochlorid, Fp: 134-135°C
46. 2-(2 -Hydroxy-3'-di-n-propylamino-propoxy)-5-methyl-β-(4-chlorphenyl)-propiophenon-hydrochlorid Fp: 119-123°C
47. 2-(2'-Hydroxy-3'-di-n-propylamino-propoxy)-5-chlor-β-(p-fluorphenyl)-propiophenon-hydrochlorid, Fp: 139-144oC
48. 2-(2'-Hydroxy-3'-n-propylamino-propoxy)-5-methyl-β-(3,4-dimethoxyphenyl)-propiophenon-hydrochlorid, Fp: 132-133°C
49. 2-(2'-Hydroxy-3'-n-propylaminopropoxy)-β-(3,4,5-tri-methoxyphenyl)-propiophenon, Fp: 197-198°C
50. 2-[2'-Hydroxy-3'-(2-hydroxyethylamino)-propoxy]-5-chlor-β-phenyl-propiophenon, Fp: 99-103°C
51. 2-(2'-Hydroxy-3'-aminopropoxy)-β-phenyl-propiophenon-oxalat Fp: 149-151°C
52. 2-(2'-Hydroxy-3'-methylaminopropoxy)-β-phenyl-propio-phenon-hydrochlorid, Fp: 141-143°C
53. 2-(2'-Hydroxy-3'-ethylamino-propoxy)-β-phenyl-propio-phenon-hydrochlorid, Fp: 162-164°C
54. 2-(2'-Hydroxy-3'-n-propylamino-propoxy)-4-methoxy-β-phenyl-propiophenon-hydrochlorid, Fp: 174-177°C

**Beispiel 55**

2-(2'-Hydroxy-3'-piperidino-propoxy)-β-(4-dimethylamino-phenyl)-propiophenon

8 g 2-(2',3'-Epoxypropoxy)-β-(4-dimethylaminophenyl)-pro-piophenon (0,025 Mol) und 20 ml Piperidin wurden in 100 ml Isopropanol gelöst und die Mischung 6 h lang auf dem Wasserbad gehalten. Anschließend wurden das Lösungsmittel und überschüssiges Amin unter vermindertem Druck abdestil-liert. Es verblieben 9 g öliger Rückstand, der aus wenig absolutem Ethanol unter Zusatz von Aktivkohle kristalli-siert wurde. Ausbeute: 3,15 g (31 %), Fp: 113-115°C.

**Beispiel 56**

2-(2'-Hydroxy-3'-isopropylamino-propoxy)-β-(4-dimethyl-aminophenyl)-propiophenon-hydrochlorid

8 g 2-(2',3'-Epoxypropoxy)-β-(4-dimethylamino-phenyl)-propiophenon und 13 ml Isopropylamin in Isopropanol wurden analog Beispiel 55 umgesetzt. Der verbleibende Rückstand nach der üblichen Aufarbeitung wurde in 150 ml Aceton gelöst und mit 4,6 ml etherischer HCl (etwa 5 N) versetzt. Das Kristallisat wurde abgesaugt und nochmals aus Aceton umkristallisiert. Ausbeute: 3,8 g (36,5 %), Fp: 149°C.

Analog wurden hergestellt:

57. 2-(2'-Hydroxy-3'-cyclopropylamino-propoxy)-β-(4-di-methylaminophenyl)-propiophenon-hydrochlorid, Fp: 128-133°C

58. 2-(2'-Hydroxy-3'-n-butylamino-propoxy)-β-(4-dimethyl-aminophenyl)-propiophenon-hydrochlorid, Fp: 161-163°C

59. 2-[2'-Hydroxy-3'-(2-hydroxyethylamino)-propoxy]-β-(di-methylaminophenyl)-propiophenon-hydrogenoxalat, Fp: 138°C

60. 2-[2'-Hydroxy-3'-(3-methoxy-prop-2-ylamino)-propoxy]-β-(4-dimethyla-minophenyl)-propiophenon-hydrochlorid, Fp: 134-135°C

61. 2-(2'-Hydroxy-3'-n-propylämino-pr-opoxy)-β-(4-diethyl-aminophenyl)-propiophenon-hydrochlorid, Fp: 154-155°C

62. 2-(2'-Hydroxy-3'-n-propylamino-propoxy)-5-chlor-β-(4-dimethylaminophenyl)-propiophenon-hydrochlorid, Fp: 166-167°C

63. 2-(2'-Hydroxy-3'-n-propylamino-propoxy)-5-methyl-β-(4-dimethylaminophenyl)-propiophenon-hydrochlorid, Fp: 161-162°C

64. 2-[2'-Hydroxy-3'-(N,N-diisopropylamino)-propoxy]-β-(4-dimethylaminophenyl)-propiophenon-hydrochlorid, Fp: 143=-144°C

65. 2-[2'-Hydroxy-3'-(N,N-diisopropylamino)-propoxy]-5-methyl-β-(4-dimethylaminophenyl)-propiophenon-hydro-chlorid, Fp: 143-145°C

66. 2-[2'-Hydroxy-3'-(N,N-diisopropylamino)-propoxy]-5-chlor-β-(4-dimethylaminophen-yl)-propiophenon-hydro-chlorid, Fp: 148-150°C

67. 2-[2'-Hydroxy-3'-(4-methyl-1-piperazinyl)-propoxy]-β-(4-dimethylaminophenyl)-propiophenon-hydrochlorid, Fp:129°C

68. 2-[2'-Hydroxy-3'-(4-methyl-1-piperazinyl)-propoxy]-5-methyl-β-(4-dimethylaminophenyl)-propiophenon-hydro-chlorid, Fp: 169-170°C

69. 2-[2'-Hydroxy-3'-(3-methoxy-prop-2-ylamino)-propoxy]-5-methyl-β-(4-dimethylaminophenyl)-propiophenon-hydro-chlorid, Fp: 121-124°C

**Beispiel 70**

2-(2'-Hydroxy-3'-n-propylamino-propoxy)-4-hydroxy-β-phenyl-propiophenon-hydrochlorid

Das gemäß Beispiel V erhaltene Produkt wurde mit 1 g Pd/C (5 %) versetzt und in 150 ml Methanol gelöst. Anschließend wurde hydriert (Wasserstoffaufnahme 1830 ml). Nach Beendi-gung der Wasserstoffaufnahme wurde der Katalysator abfil-triert und das Lösungsmittel abdestilliert. Es wurden 15,8 g rotes zähes Öl erhalten, das 1 h mit 100 ml 1 N Salzsäure am Rückfluß erhitzt wurde. Das Öl, welches sich nicht löste, wurde abgetrennt. Die nach dem Abkühlen entstandenen Kristalle wurden abgesaugt und aus 150 ml Ethanol/Aceton (1 : 2) 2 x umkristallisiert. Ausbeute 2,7 g; Fp: 146-148°C.

Analog wurde hergestellt:

71. 2-(2'-Hydroxy-3'-n-propylamino-propoxy)-5-hydroxy-β-phenyl-propiophenon-hydrochlorid, Fp: 220-221°C

**Patentansprüche** für die Vertragsstaaten BE, CH, DE, FR, GB, IT, LI, NL, SE

1. Aminopropanolderivate der Formel I

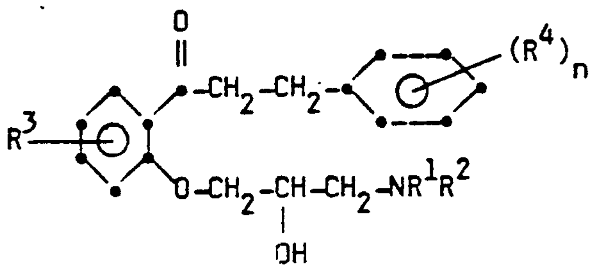

I

.

in der

$R^1$ und $R^2$ gleich oder verschieden sind und Wasserstoff-atome, Alkyl- Cycloalkyl- Alkenyl- ,Alkinyl- oder Hydroxyalkylreste mit jeweils bis zu 6 C-Atomen, Alkoxyalkyl-, Alkylthioalkyl- oder Dialkylaminoalkyl-reste mit jeweils bis zu 9 C-Atomen oder Phenylalkyl-oder Phenoxyalkylreste mit bis zu 6 C-Atomen im Alkyl und gegebenenfalls im Phenylrest durch Alkyl oder Alkoxy mit jeweils bis zu 3 C-Atomen substituiert bedeuten oder

$R^1$ und $R^2$ zusammen mit dem sie verbindenden Stickstoff-atom einen 5 bis 7-gliedrigen gesättigten heterocycli-schen Ring bilden, der gegebenenfalls durch einen oder zwei Phenyl- und/oder Hydroxyreste substituiert sein und ein Sauerstoff- oder Stickstoffatom als weiteres Heteroatom im Ring enthalten kann, wobei ein zusätz-liches Stickstoffatom durch einen Alkylrest mit 1 bis 3 C-Atomen oder einen Phenylrest substituiert

sein kann,

$R^3$ ein Wasserstoffatom, einen Alkylrest mit bis zu 3 C-Atomen, ein Fluor-, Chlor- oder Bromatom, eine Hydroxygruppe oder eine Alkoxygruppe mit bis zu 6 C-Atomen ist,

$R^4$ ein Wasserstoffatom, einen Alkylrest mit bis zu 3 C-Atomen, ein Fluor-, Chlor- oder Bromatom, einen Alkoxyrest mit bis zu 3 C-Atomen oder den Rest $NR^5R^6$ bedeutet, worin $R^5$ und $R^6$ gleich oder verschieden sind und Alkylreste mit bis zu 6 C-Atomen oder zusammen mit dem sie verbindenden Stickstoffatom einen heterocyclischen Ring darstellen, ist, wobei jedoch die Reste $R^1$, $R^3$ und $R^4$ nicht gleichzeitig Wasserstoffatome sein dürfen, wenn $R^2$ einen Alkylrest mit 3 bis 5C-Atomen bedeutet, und n die Zahl 1, 2 oder 3 ist, und ihre physiologisch verträglichen Säureadditions-salze.

2. Aminopropanolderivate der Formel I gemäß Anspruch 1, dadurch gekennzeichnet, daß $R^3$ ein Wasserstoffatom ist.

3. Aminopropanolderivate der Formel I gemäß Anspruch 1, dadurch gekennzeichnet, daß n 1 oder 2 ist.

4. Aminopropanolderivate der Formel I gemäß Anspruch 1, dadurch gekennzeichnet, daß die Gruppe $NR^1R^2$ ein Piperidin-, Piperazin-, N-Methylpiperazin-, Morpholin-oder Diisopropylaminorest ist.

5. Aminopropanolderivate der Formel I gemäß Anspruch 1, dadurch gekennzeichnet, daß

$R^1$ und $R^2$ zusammen mit dem sie verbindenden Stick-stoffatom ein Piperidin-, Piperazin-, N-Methyl-piperazin-, Morpholin- oder Diisopropylaminorest,

$R^3$ ein Wasserstoffatom und

n die Zahl 1 oder 2 sind.

6. Verfahren zur Herstellung der Aminopropanolderivate der Formel I nach Anspruch 1, dadurch gekennzeichnet, daß man

a) eine Verbindung der Formel II

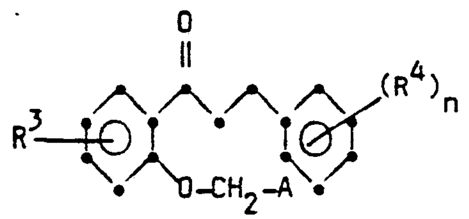

II

in der A den Rest 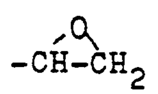 oder 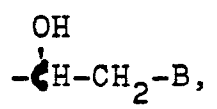

wobei B für eine nucleofuge Abgangsgruppe steht, bedeuten und $R^3$, $R^4$ und n die angegebenen Bedeutungen haben, mit einem Amin der Formel III .

$HNR^1R^2$ (III),

in der $R^1$ und $R^2$ die angegebenen Bedeutungen haben, umsetzt oder

b) eine Verbindung der Formel IV

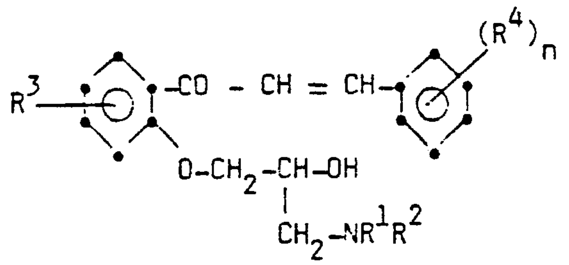

IV

worin $R^1$ , $R^2$ , $R^3$, $R^4$ und n die angegebene Bedeu-tung besitzen, katalytisch hydriert, und gege-benenfalls die so erhaltenen Verbindungen in ihre Säureadditionssalze physiologisch verträglicher Säuren überführt.

7. Therapeutische Mittel, enthaltend eine Verbindung der Formel I.

8. Verbindung der Formel I gemäß Anspruch 1 zur Verwen-dung bei der Bekämpfung von Krankheiten.

**Patentansprüche** für den Vertragsstaat AT

1. Verfahren zur Herstellung von Aminopropanolderivaten der Formel I

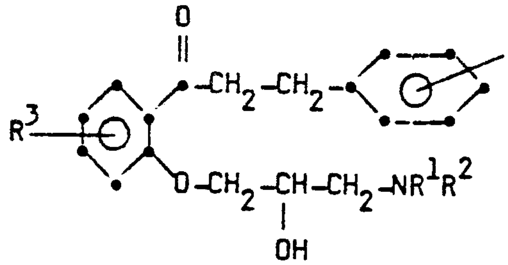

I

in der

$R^1$ und $R^2$ gleich oder verschieden sind und Wasserstoff-atome, Alkyl-, Cycloalkyl- Alkenyl-, Alkinyl- oder Hydroxyalkylreste mit jeweils bis zu 6 C-Atomen, Alkoxyalkyl-, Alkylthioalkyl- oder Dialkylaminoalkyl-reste mit jeweils bis zu 9 C-Atomen oder Phenylalkyl-oder Phenoxyalkylreste mit bis zu 6 C-Atomen im Alkyl und gegebenenfalls im Phenylrest durch Alkyl oder Alkoxy mit jeweils bis zu 3 C-Atomen substituiert bedeuten oder

$R^1$ und $R^2$ zusammen mit dem sie verbindenden Stickstoff-atom einen 5 bis 7-gliedrigen gesättigten heterocycli-schen Ring bilden, der gegebenenfalls durch einen oder zwei Phenyl- und/oder Hydroxyreste substituiert sein und ein Sauerstoff- oder Stickstoffatom als weiteres Heteroatom im Ring enthalten kann, wobei ein zusätz-liches Stickstoffatom durch einen Alkylrest mit 1 bis 3 C-Atomen oder einen Phenylrest substituiert sein kann,

$R^3$ ein Wasserstoffatom, einen Alkylrest mit bis zu 3 C-Atomen, ein Fluor-, Chlor- oder Bromatom, eine Hydroxygruppe oder eine Alkoxygruppe mit bis zu 6 C-Atomen ist,

$R^4$ ein Wasserstoffatom, einen Alkylrest mit bis zu 3 C-Atomen, ein Fluor-, Chlor- oder Bromatom, einen Alkoxyrest mit bis zu 3 C-Atomen oder den Rest $NR^5R^6$ bedeutet, worin $R^5$ und $R^6$ gleich oder verschieden sind und Alkylreste mit bis zu 6 C-Atomen oder zusammen mit dem sie verbindenden Stickstoffatom einen heterocyclischen Ring darstellen, ist, wobei jedoch die Reste $R^1$, $R^3$ und $R^4$ nicht gleichzeitig Wasserstoffatome sein dürfen, wenn $R^2$ einen Alkylrest mit 3 bis 5C-Atomen bedeutet, und n die Zahl 1, 2 oder 3 ist, sowie deren physiologisch verträglichen Säureadditions-salzen, <u>dadurch gekennzeichnet</u>, daß man

a) eine Verbindung der Formel II

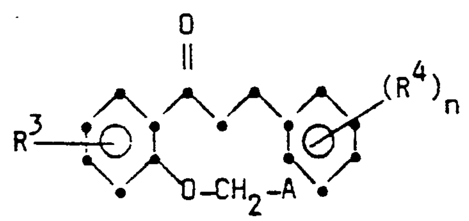

II

in der A den Rest 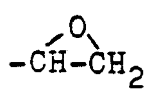 oder 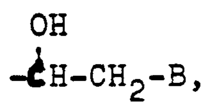 wobei B

wobei B für eine nucleofuge Abgangsgruppe steht, bedeuten und $R^3$, $R^4$ und n die angegebenen Bedeutungen haben, mit einem Amin der Formel III

$HNR^1R^2$ (III),

in der $R^1$ und $R^2$ die angegebenen Bedeutungen haben, umsetzt oder

b) eine Verbindung der Formel IV

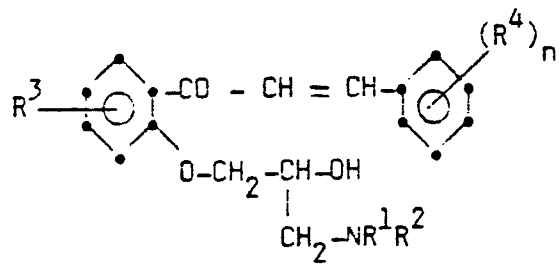

IV

worin-R [1], $R^2$, $R^3$, $R^4$ und n die angegebene Bedeu-tung besitzen, katalytisch hydriert, und gege-benenfalls die so erhaltenen Verbindungen in ihre Säureadditionssalze physiologisch verträglicher Säuren überführt.

2. Verfahren gemäß Anspruch 1, <u>dadurch gekennzeichnet</u>, daß man Verbindungen der Formel I herstellt, in denen $R^3$ ein Wasserstoffatom darstellt.

3. Verfahren gemäß Anspruch 1, <u>dadurch gekennzeichnet</u>, daß man Verbindungen der Formel I herstellt, in

denen n 1 oder 2 ist.

4. Verfahren gemäß Anspruch 1, dadurch gekennzeichnet, daß man Verbindungen der Formel I herstellt, in denen die Gruppe $NR^1R^2$ ein Piperidin-, Piperazin-, N-Methyl-piperazin-, Morpholin- oder Diisopropylaminorest ist.

5. Verfahren gemäß Anspruch 1, dadurch gekennzeichnet, daß man Verbindungen der Formel I herstellt, in denen

$R^1$ und $R^2$ zusammen mit dem sie verbindenden Stick-stoffatom ein Piperidin-, Piperazin-, N-Methyl-piperazin-, Morpholin- oder Diisorpoylaminorest,

$R^3$ ein Wasserstoffatom und

n die Zahl 1 oder 2

sind.

**Claims** for the contracting States : BE, CH, DE, FR, GB, IT, LI, NL, SE

1. An aminopropanol derivative of the formula I

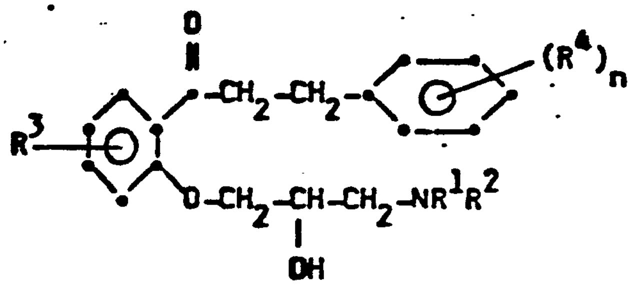

I,

where

$R^1$ and $R^2$ are identical or different and each is hydrogen, or alkyl, cycloalkyl, alkenyl, Alkynyl or hydroxyalkyl each having up to 6 carbon atoms, alkoxyalkyl, alkylthio-alkyl or dialkylaminoalkyl each having up to 9 carbon atoms, or phenylalkyl or phenoxyalkyl, where alkyl is of up to 6 carbon atoms and the phenyl is unsubstituted or substituted by alkyl or alkoxy each haVing up to 3 carbon atoms, or

$R^1$ and $R^2$, together with the nitrogen atom linking them, are a 5- to 7-membered saturated heterocyclic ring, which is unsubstituted or substituted by one or two phenyl and/ or hydroxyl radicals and may contain oxygen or nitrogen as a further hetero-atom in the ring, in which case an additional nitrogen atom may be substituted by alkyl of 1 to 3 carbon atoms or phenyl,

$R^3$ is hydrogen, alkyl of up to 3 carbon atoms, fluorine, chlorine, bromine, hydroxyl or alkoxy of up to 6 carbon atoms,

$R^4$ is hydrogen, alkyl of up to 3 carbon atoms, fluorine, chlorine, bromine, alkoxy of up to 3 carbon atoms or $NR^5R^6$, where $R^5$ and $R^6$ are identical or different and each is alkyl of up to 6 carbon atoms, or $R^5$ and $R^6$, together with the nitrogen atom linking them, are a heterocyclic ring, but where $R^1$, $R^3$ and $R.^4$ cannot all be hydrogen if $R^2$ is alkyl of 3 to 5 carbon atoms, and

n is 1, 2 or 3,

and its physioldgically acceptable acid addition salts.

2. An aminopropanol derivative of the formula I as claimed in claim 1, wherein $R^3$ is hydrogen.

3. An aminopropanol derivative of the formula I as claimed in claim 1, wherein n is 1 or 2.

4. An aminopropanol derivative of the formula I as claimed in claim 1, wherein $NR^1R^2$ is a piperidine, piperazine, N-methylpiperazine, morpholine or diisopropylamino radical.

5. An aminopropanol derivative of the formula I as claimed in claim 1, wherein

$R^1$ and $R^2$, together with the nitrogen linking them are a piperidine, piperazine, N-methylpiperazine, morpholine or diisopropylamino radical, $R^3$ is hydrogen, and n is 1 or 2.

6. A process for the preparation of an aminopropanol derivative of the formula 1 as claimed in claim 1, wherein

a) a compound of the formula II

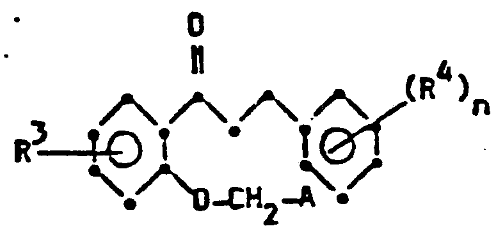

II,

where A is

FIG23/10
or

FIG24/10
where B is a nucleofugic leaving group, and $R^3$ $R^4$ and n have the above meanings is reacted with an amine of the formula III
$HNR^1R^2$ III,
where $R^1$ and $R^2$ have the above meanings, or
b) a compound of the formula IV

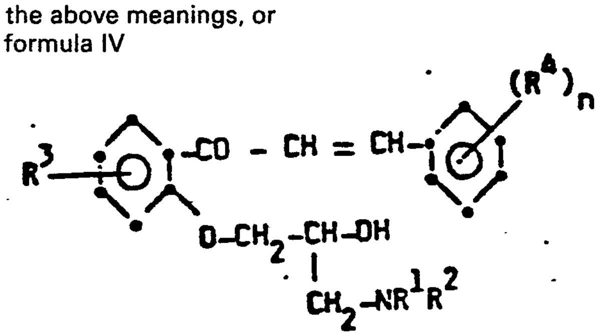
IV,

FIG25/35

where $R^1$, $R^2$, $R^3$, $R^4$ and n have the above meanings, is subjected to catalytic hydrogenation, and, if desired, the compound thus obtained is converted into an acid addition salt of a physiologically acceptable acid.
7. A therapeutic agent containing a compound of the formula I.
8. A compound of the formula I as claimed in claim 1, for use in combating diseases.

**Claims** for the contracting State :AT

1. A process for the preparation of an aminopropanol derivative of the formula I

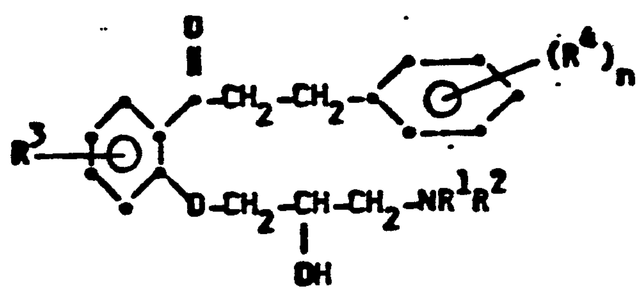
I,

where
$R^1$ and $R^2$ are identical or different and each is hydrogen, or alkyl, cycloalkyl, alkenyl, alkynyl or hydroxyalkyl each having up to 6 carbon atoms, alkoxyalkyl, alkylthioalkyl or dialkylaminoalkyl each having up to 9 carbon atoms, or phenylalkyl or phenoxyalkyl, where alkyl is of up to 6 carbon atoms and the phenyl is unsubstituted or substituted by alkyl or alkoxy each having up to 3 carbon atoms, or $R^1$ and $R^2$, together with the nitrogen atom linking them, are a 5-to 7-membered saturated heterocyclic ring, which is un-substituted or substituted by one or two phenyl and/or hy-droxyl radicals and may contain oxygen or nitrogen as a further hetero-atom in the ring, in which case an additional nitrogen atom may be substituted by alkyl of 1 to 3 carbon atoms or phenyl, $R^3$ is hydrogen, alkyl of up to 3 carbon atoms, fluorine, chlorine, bromine, hydroxyl or alkoxy of up to 6 carbon atoms, $R^4$ is hydrogen, alkyl of up to 3 carbon atoms, fluorine, chlorine, bromine, alkoxy of up to 3 carbon atoms or $NR^5R^6$, where $R^5$ and $R^6$ are identical or different and each is alkyl of up to 6 carbon atoms, or $R^5$ and $R^6$, together with the nitrogen atom linking them, are a heterocyclic ring, but where $R^1$, $R^3$ and $R^4$ cannot all be hydrogen if $R^2$ is alkyl of 3 to carbon atoms, and n is 1,2 or 3, and its physiologicylly acceptable acid addition salts, wherein
a) a compound of the formula II

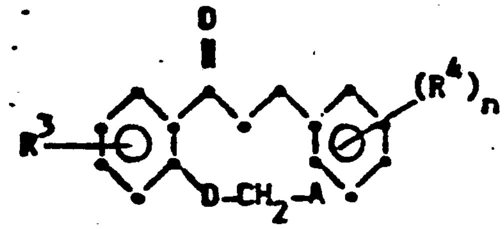

II,

where A is 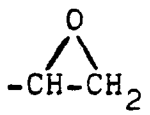 or 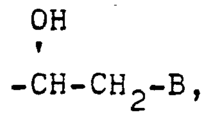,

where B is a nucleofugic leaving group, and $R^3$, $R^4$ and n have the above meanings, is reacted with an amine of the formula III

$HNR^1R^2$ KKK,

where $R^1$ and $R^2$ have the above meanings, or

b) a compound of the formula IV

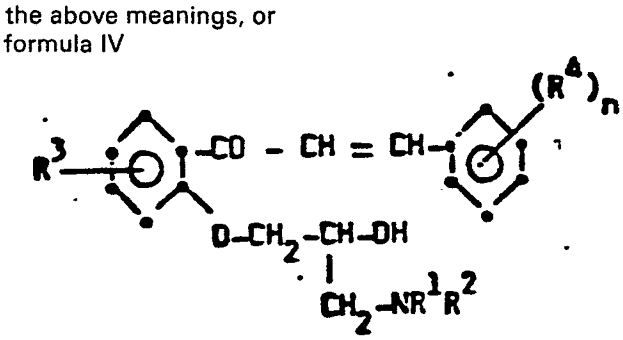

IV,

where $R^1$, $R^2$, $R^3$, $R^4$ and n have the above meanings, is subjected to catalytic hydrogenation, and, if desired, the compound thus obtained is converted into an acid addition salt of a physiologically acceptable acid.

2. A process as clamed in clam 1, wheren a compound of the formula I, where $R^3$ is hydrogen, is prepared.

3. A process as claimed in claim 1, wherein a compound of the formula I, where n is 1 or 2, is prepared.

4. A process as claimed in claim 1, wherein a compound of the formula I, where $NR^1R^2$ is a piperidine, piperazine, N-methylpiperazine, morpholine or diisopropylamino radical, is prepared.

5. A.process as claimed in claim 1, wherein a compound of the formula I, where

$R^1$ and $R^2$, together with the nitrogen linking them, are a piperidine, piperazine, N-methylpiperazine, morpholine or diisopropylamino redical, $R^2$ is hydrogen, and n is 1 or 2, is prepared.

**Revendications** pour les Etats contractants: BE, CH, DE, FR, GB, IT, LI, NL, SE

1. Dérivés d'aminopropanol de formule 1

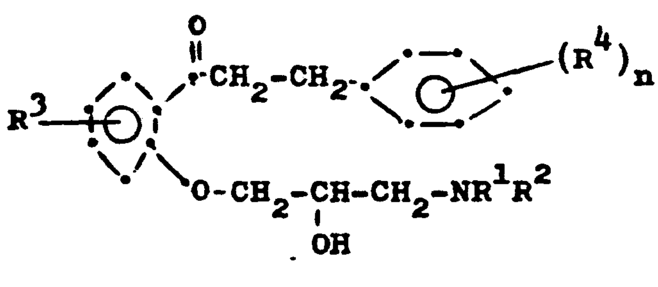

I

dans laquelle

$R^1$ et $R^2$ sont ide ıtiques ou différents et représentent des atomes d'hydrogène, des restes alky!e, cycloalkyle, alcényle, alcynyle ou hydroxyalkyle ayant chacun jusqu'à 6 atomes C, des restes alcoxyalkyle, alkylthioalkyle ou dialkylaminoalkyle ayant chacun jusqu'à 9 atomes C, ou des restes phénylalkyle ou phénoxyalkyle ayant jusqu'à 6 atomes C dans l'alkyle et éventuellement substitués dans le reste phényle par alkyle ou alcoxy ayant chacun jusqu,à 3 atomes C, ou $R^1$ et $R^2$ représentent, ensemble avec l'atome d'azote qui les relie, un hétérocycle saturé à 5 à 7 termes, qui peut être substitué par un ou deux restes phényle et/ou hydroxy et contenir dans le cycle un atome d'oxygène ou d'azote comme hétéroatome supplémentaire, un atome d'azote supplé-mentaire pouvant être substitué par un reste alkyle à 1 ou 3 atomes C ou par un reste phényle, $R^3$ représente un atome

d'hydrogène, un reste alkyle ayant jusqu'à 3 atomes C, un atome de fluor, chlore ou brome, un groupe hydroxy ou un groupe alcoxy ayant jusqu'à 6 atomes C, $R^4$ représente un atome d'hydrogène, un reste alkyle ayant jusqu'à 3 atomes C, un atome de fluor, chlore ou brome, un reste alcoxy ayant jusqu'à 3 atomes C ou le reste $NR^5R^6$ dans lequel $R^5$ et $R^6$ sont identiques ou différents et représentent des restes alkyle ayant jusqu'à 6 atomes C ou forment, ensemble avec l'atome d'azote qui les relie, un hétérocycle, les restes $R^1$, $R^3$ et $R^4$ ne devant toutefois pas désigner simultanément des atomes d'hydrogène lorsque $R^2$ représente un reste alkyle à 3 à 5 atomes C, et n représente un des nombres 1, 2 et 3, ainsi que leurs sels d'addition d'acide physiologiquement tolérés.

2. Dérivés d'aminopropanol de formule I, selon la revendi-cation 1, caractérisés par le fait que $R^3$ représente un atome d'hydrogène.

3. Dérivés d'aminopropanol de formule I, selon la revendi-cation 1, caractérisés par le fait que n représente 1 ou 2.

4. Dérivés d'aminopropanol de formule I, selon la revendi-cation 1, caractérisés par le fait que le groupe $NR^1R^2$ est un reste pipéridine, pipérazine, N-méthylpipérazine, morpholine ou diisopropylamino.

5. Dérivés d'aminopropanol de formule I, selon la revendi-cation 1, caractérisés par le fait que

$R^1$ et $R^2$, ensemble avec l'atome d'azote qui les relie, forment un reste pipéridine, pipérazine, N-méthylpipérazine, morpholine ou diisopropylamino,

$R^3$ représente un atome d'hydrogène et n est le nombre 1 ou 2.

6. Procédé de préparation des dérivés d'aminopropanol de formule I, selon la revendication 1, caractérisé par le fait que

a) on fait réagir un composé de formule II

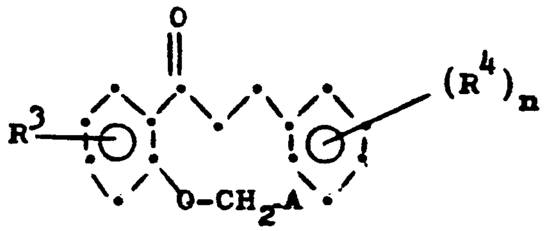

II

dans laquelle A represente le reste 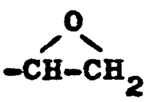 ou 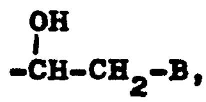

B étant mis à la placé d'un groupe de départ nucléofuge, et $R^3$, $R^4$ et n ont les significations données plus haut, avec une amine de formule III

$HNR^1R^2$ III

dans laquelle $R^1$ et $R^2$ ont les significations données plus haut, ou

b) on soumet à une hydrogénation catalytique un composé de formule IV

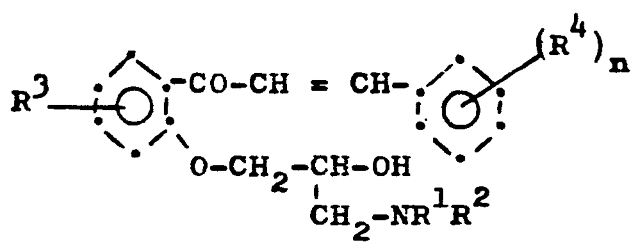

IV

dans laquelle $R^1$, $R^2$, $R^3$, $R^4$ et n ont les significations données plus haut, et, éventuellement, on transforme les composés ainsi obtenus en leurs sels d'addition d'acide physiologiquement tolerés.

7. Agents thérapeutiques contenant un composé de formule I.

8. Composé de formule I, selon la revendication 1, pour l'utilisation dans la lutte contre les maladies.

**Revendications** pour l'Etat contractant: AT

1. Procédé de préparation de dérivés d'aminopropanol de formule I

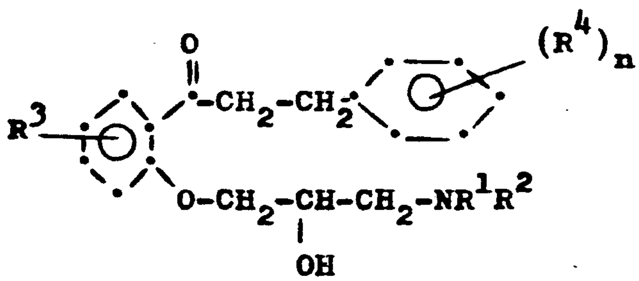

I

dans laquelle

$R^1$ et $R^2$ sont identiques ou différents et représentent des atomes d'hydrogène, des restes alkyle, cycloalkyle, alcényle ou hydroxyalkyle ayant chacun jusqu'à 6 atomes C, des res-tes alcoxyalkyle, alkylthioalkyle ou dialkylaminoalkyle ayant chacun jusqu'à 9 atomes C, ou des restes phénylalkyle ou phénoxyalkyle ayant jusqu'à 6 atomes C dans l'alkyle et éventuellement substitués dans le reste phényle par alkyle ou alcoxy ayant chacun jusqu'à 3 atomes C, ou $R^1$ et $R^2$ représentent, ensemble avec l'atome d'azote qui les relie, un hétérocycle saturé à 5 à 7 termes, qui peut être substitué par un ou deux restes phényle et/ou hydroxy et contenir dans le cycle un atome d'oxygène ou d'azote comme hétéroatome supplémentaire, un atome d'azote supplé-mentaire pouvant être substitué par un reste alkyle à 1 ou 3 atomes C ou par un reste phényle, $R^3$ représente un atome d'hydrogène, un reste alkyle ayant jusqu'à 3 atomes C, un atome de fluor, chlore ou brome, un groupe hydroxy ou un groupe alcoxy ayant jusqu'à 6 atomes C, $R^4$ représente un atome d'hydrogène, un reste alkyle ayant jusqu'à 3 atomes C, un atome de fluor, chlore ou brome, un reste alcoxy ayant jusqu'à 3 atomes C ou le reste $NR^5R^6$, dans lequel $R^5$ et $R^6$ sont identiques ou différents et représentent des restes alkyle ayant jusqu'à 6 atomes C ou forment, ensemble avec l'atome d'azote qui les relie, un hétérocycle, les restes $R^1$, $R^3$ et $R^4$ ne devant toute-fois pas désigner simultanément des atomes d'hydrogène lorsque $R^2$ représente un reste alkyle à 3 à 5 atomes C, et n représente un des nombres 1, 2 et 3, ainsi que leurs sels d'addition d'acide physiologiquement tolérés, caractérisé par le fait que

a) on fait réagir un composé de formule II

FIG36/30

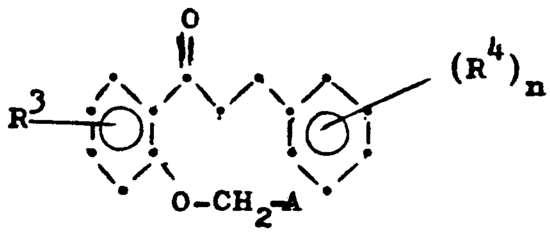

II

dans laquelle A représente le reste 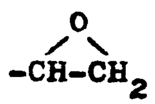 ou 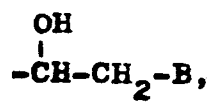

B étant mis à la placé d'un groupe de départ nucléofuge, et $R^3$, $R^4$ et n ont les significations données plus haut, avec une amine de formule III

$HNR^1R^2$ III

dans laquelle $R^1$ et $R^2$ ont les significations données plus haut, ou

b) on soumet à une hydrogénation catalytique un composé de formule IV

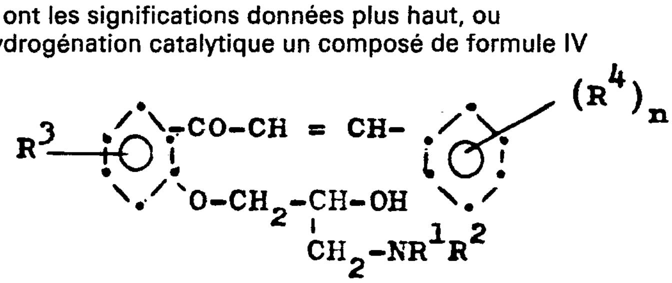

IV

dans laquelle $R^1$, $R^2$, $R^3$, $R^4$ et n ont les significations données plus haut, et, éventuellement, on transforme les composés ainsi obtenus en leurs sels d'addition d'acide physiologiquement tolérés.

2. Procédé selon la revendication 1, caractérisé par le fait qu'on prépare des composés de formule I, dans lesquels $R^3$ représente un atome d'hydrogène.

3. Procédé selon la revendication 1, caractérisé par le fait qu'on prépare des composés de formule I, dans lesquels n représente 1 ou 2.

4. Procédé selon la revendication 1, caractérisé par le fait qu'on prépare des composés de formule I, dans lesquels le groupe $NR^1R^2$ est un reste pipéridine, pipérazine, N-méthylpipérazine, morpholine ou diisopropylamino.

5. Procédé selon la revendication 1, caractérisé par le fait qu'on prépare des composés de formule I, dans lesquels $R^1$ et $R^2$, ensemble avec l'atome d'azote qui les relie, forment un reste pipéridine, pipérazine, N-méthylpipéra-zine, morpholine ou diisopropylamino, $R^3$ représente un atome d'hydrogène et n est le nombre 1 ou 2.